# FASCICULE DE BREVET EUROPEEN

(11) **EP 0 595 948 B1**
(45) Date de publication et mention de la délivrance du brevet: **21.05.1997**
(21) Numéro de dépôt: 92916070.3
(22) Date de dépôt: 15.07.1992
(51) Int. Cl.: C12N 15/81, C12N 1/19, C12N 15/90, C12N 15/53

(54) **SOUCHES DE LEVURE AVEC INTEGRATION STABLE DE GENES HETEROLOGUES**
Hefe Stämme mit stabiler Integration heterologer Gene
YEAST STRAINS WITH STABLE INTEGRATION OF HETEROLOGOUS GENES

(30) Priorité: 15.07.1991 FR 9108884
(43) Date de publication de la demande: 11.05.1994
(73) Titulaire: CENTRE NATIONAL DE LA RECHERCHE SCIENTIFIQUE (CNRS), 75794 Paris Cédex 16 (FR)
(72) Inventeur: POMPON, Denis 9, place du Marché-neuf, F-91190 Gif-sur-Yvette (FR); CULLIN, Christophe, F-92150 Antony (FR); TRUAN, Gilles, F-91940 Les Ulis (FR); URBAN, Philippe, F-92220 Bagneux (FR); SLONIMSKI, Piotr Résidence du CNRS, Bât. ABC, F-91190 Gif-sur-Yvette (FR)
(74) Mandataire: Warcoin, Jacques
(86) Numéro de dépôt international: FR9200682
(87) Numéro de publication internationale: WO9302200

(56) Documents cités:
- EP-A- 0 260 160
- EP-A- 0 399 455
- WO-A-86/07091
- GENE vol. 65, 1988, AMSTERDAM, THE NETHERLANDS pages 203 - 217 C. CULLIN AND D. POMPON 'Synthesis of functional mouse cytochromes P-450 P1 and chimeric P-450 P3-1 in the yeast Saccharomyces cerevisiae'

## Description

La présente invention concerne une souche de levure dans les chromosomes de laquelle sont intégrés de façon stable plusieurs gènes hétérologues. La présente invention concerne en outre un procédé d'expression chez la levure d'une fonction complexe liée à l'activité de plusieurs facteurs hétérologues, et en particulier un procédé d'expression chez la levure de l'activité monooxygénase de cytochrome(s) P450 hétérologue(s).

Les cytochromes P450 (ci-après abrégés P450) constituent une superfamille d'enzymes membranaires ayant des activités de type monooxygénase très variées. Leurs activités sont susceptibles d'être utilisées dans une large gamme de domaines d'application. On peut citer à titre d'exemples non limitatifs :
- la bioconversion d'un nombre quasi-illimité de molécules lipophiles par des réactions d'insertion d'un atome d'oxygène, suivies ou non de réarrangements dans des liaisons carbone-carbone ou carbone-hydrogène, et par l'addition d'oxygène sur des hétéroatomes variés (soufre, azote, phosphore). Le P450 utilise l'oxygène de l'air comme oxydant.
- le diagnostic in vitro de la formation de métabolites toxiques ou mutagènes par le métabolisme hépatique humain de molécules xénobiotiques naturelles ou artificielles (polluants, médicaments, additifs). Une telle prédiction est de première importance en particulier dans le cas du développement de nouvelles molécules d'intérêt pharmaceutique.
- L'identification et la destruction de molécules toxiques ou polluantes de l'environnement.

Compte tenu de ce large domaine d'application, l'expression de formes hétérologues de P450 et de leurs activités chez les microorganismes qui en sont dépourvus, est clairement digne d'intérêt. Deux difficultés sont alors apparues : premièrement, la nature membranaire des P450 d'eucaryotes rend souhaitable l'utilisation d'un microorganisme hôte de type eucaryote, d'où le choix des levures ; deuxièmement, ces enzymes ne sont fonctionnels qu'en présence "d'enzymes associés" qui jouent un rôle de transporteurs d'électrons spécifiques. Alors que la diversité des cytochromes P450 est extrême, les "enzymes REDOX associés" sont peu nombreux et comprennent le cytochrome b5, la NADH-cytochrome b5 réductase et surtout la NADPH-cytochrome P450 réductase. Par ailleurs certains enzymes dits de phase II, tels que par exemple l'époxyde hydrolase microsomale, peuvent être nécessaires au couplage métabolique entre différents P450 ou à la destruction d'intermédiaires chimiques hautement réactifs formés lors de certaines réactions comme par exemple le métabolisme des hydrocarbures polycycliques.

L'expression in vivo et à un haut niveau de cytochromes P450 hétérologues chez la levure ne conduit à de hauts niveaux d'activité que si ces "enzymes rédox associés", essentiels au fonctionnement des P450, peuvent être coexprimés dans la levure, dans des stoechiométries convenables entre eux et relativement aux P450. Des données récentes montrent que :
- un rapport molaire réductase / P450 hétérologue trop bas conduit à une faible activité spécifique du fait du défaut de réductase.
- un rapport molaire réductase / P450 hétérologue trop élevé conduit à la destruction d'une fraction importante du P450 du fait du fort accroissement du nombre de cycles catalytiques abortifs et de la production par l'excès de réductase de radicaux oxygénés dangereux pour la cellule. La conséquence est une perte de viabilité des cellules, voire une chute de l'activité due à la destruction des cytochromes P450.
- un rapport molaire cytochrome b5/P450 trop bas conduit à une activité réduite des P450 de classe B. Par ailleurs, la présence de haut niveau de cytochrome b5 semble jouer un effet protecteur par rapport aux effets toxiques liés à des niveaux excessifs de réductase;
- un rapport molaire cytochrome b5/P450 trop élevé conduit à l'inhibition de l'activité des P450 de classe A et peut potentiellement réduire la quantité d'hème intracellulaire non lié et être alors toxique pour la cellule.

Afin de résoudre ces difficultés, on a selon l'invention construit par intégration génomique de gènes synthétiques, un ensemble de souches de levure exprimant de manière stable et modulable (par la composition du milieu culture), un environnement enzymatique optimisable pour l'expression de l'activité d'un P450 hétérologue quelconque.

De nombreuses publications ont décrit l'expression de cytochromes P450 hétérologues chez la levure. Néanmoins le problème de l'optimisation de l'environnement cellulaire pour permettre une activité élevée **in vivo** n'a été que rarement abordé, les protéines produites étant souvent utilisées dans des buts analytiques ou de recherche **in vitro**.

Le problème de l'optimisation des activités a été abordé jusque là de deux façons :
- par la construction de protéines-fusion associant artificiellement sur une même chaîne polypeptidique une NADPH-cytochrome P450 réductase (homologue ou hétérologue) et le cytochrome P450 lui-même.
- par utilisation d'un vecteur portant sur le même plasmide à la fois une unité d'expression pour la P450 réductase et une unité d'expression pour un P450 hétérologue.

Ces deux systèmes, à savoir la construction de protéines-fusion (P450-P450 réductase) ou de vecteurs plasmidiques de coexpression, présentent de sérieux inconvénients :
1 - la construction de protéines-fusion (P450-réductase) est une opération longue et difficilement optimisable (sauf de manière empirique par l'approche essais-erreurs) dans l'état actuel des connaissances, au niveau du "design" moléculaire de la fusion. Ceci n'est pas a priori un défaut réel lorsque l'intérêt est focalisé sur une activité unique et qu'une recherche longue peut y être consacrée, c'est cependant une lourde tare durant un processus de développement où plusieurs types d'activité doivent être testées en un temps raisonnable afin d'ajuster l'outil au problème. Outre ce fait, ce système présente trois défauts majeurs qui lui sont inhérents :
   - le premier est de ne pas permettre un ajustement du rapport entre la quantité de la réductase et celle du P450. La réductase ayant a priori un cycle catalytique beaucoup plus rapide que le cytochrome, le rapport optimal pour l'activité n'est pas nécessairement de 1/1 et peut varier d'un P450 à l'autre, voire d'un substrat à l'autre pour le même P450.
   - le second provient du mauvais rendement de synthèse (ou d'une mauvaise stabilité) de la protéine-fusion; ceci se traduit dans la plupart des cas par une forte réduction du niveau d'expression de la protéine-fusion par rapport à celui du P450 exprimé seul. Cette diminution fait perdre en rendement la majorité, sinon la totalité du gain d'activité spécifique résultant de la fusion.
   - le troisième défaut est l'impossibilité sinon technique du moins pratique de généraliser cette approche de la fusion de plus de deux protéines.
2 - La coexpression de plusieurs protéine hétérologues à partir d'un plasmide unique, semble en première analyse une bonne solution. Néanmoins de tels plasmides de grandes tailles sont potentiellent instables génétiquement, surtout (ce qui est le cas le plus fréquent) lorsque les mêmes éléments promoteurs sont utilisés pour les différents gènes. Cette instabilité est un problème certain pour un usage industriel.

Par ailleurs les plasmides multicopies utilisés, du fait de la distribution aléatoire du nombre de copies dans chaque cellule, conduisent à des populations de cellules très hétérogènes quant au taux d'expression de chacun de ces deux enzymes (réductase et P450). L'activité monooxygénasique obtenue (qui est pratiquement la valeur utile) est alors la moyenne d'une distribution quadratique ; cette valeur ne peut, pour des raisons évidentes, qu'être inférieure à la valeur d'activité qui serait atteinte si le P450 (même sur un plasmide multicopies) était exprimé dans un contexte cellulaire ayant un niveau homogène et élevé de réductase.

Selon la présente invention le procédé d'expression chez la levure de l'activité monooxygénase de cytochromes P450 hétérologues est caractérisé en ce que l'on transforme avec un plasmide portant une cassette d'expression du gène du cytochrome P450 hétérologue, une souche de levure dans le génome de laquelle sont intégrés les gènes de la NADPH-cytochrome P450-réductase et du cytochrome b5 et qui co-expriment donc la NADPH réductase et le cytochrome b5.

La stabilité des souches de levure qui expriment un contexte adapté à l'expression de P450 hétérologues est un point essentiel compte tenu du caractère généralement toxique possible pour la cellule de l'expression d'activité de P450 hétérologues et de leurs enzymes associés. Ce problème prend en effet une importance critique lorsque de nombreux gènes etrangers doivent être simultanément intégrés.

Les principales instabilités génétiques prévisibles qui conduisent à la disparition ou à la modification d'activités hétérologues introduites dans des souches de levure portant un ou plusieurs gènes étrangers, sont :
(a)- l'inactivation par mutation d'un des gènes hétérologues. Un tel événement est généralement sélectionné spontanément du fait de la toxicité pour la cellule des gènes hétérologues ou du moins des activités associées.
(b)- la délétion par recombinaison du gène hétérologue lors de la duplication du gène sauvage (conversion) au sein d'un locus hétérozygote (voir schéma 1). L'avantage sélectif est double puisque il a un effet à la fois identique à (a) et un effet qui conduit à un contexte homozygote sauvage. 
Schéma 1 :
....XXX....YYYY....ZZZ → ....XXX....YYYY....ZZZ...
....XXX.....hhhhh....ZZZ → ....XXX....YYYY.....ZZZ...
   - XXX et ZZZ: : séquences intergénétiques sauvages
   - hhhhhh: : gène hétérologue
   - YYYYY: : gène sauvage cible de l'intégration
(c)- l'élimination d'un des gènes hétérologues, ou l'évolution de caractéristiques importantes de la souche (liée au réarrangement de gènes hétérozygotes endogènes inconnus non liés directement aux gènes hétérologues, mais de fonctions significatives pour les activités d'intérêt) par une recombinaison méiotique qui surviendrait au niveau des loci hétérozygotes et qui serait suivie de sporulations puis de croisements spontanés.

La prise en compte de ces mécanismes a conduit à définir les règles de stabilité pour la construction de souches multi-intégrées suivant l'invention.

Dans son aspect le plus général, la présente invention a pour objet une souche de levure dans le génome chromosomique de laquelle sont intégrés de façon stable plusieurs gènes hétérologues et permettant leur expression caractérisée en ce que :
-1- la souche de levure est une souche diploïde dont les allèles sont isogéniques à l'exception des loci qui portent les gènes hétérologues et du locus sexuel, qui sont hétérozygotes,
-2- les loci hétérozygotes sont dépourvus d'allèle portant un gène sauvage, et
-3- les gènes hétérologues sont placés sous le contrôle d'un promoteur inductible et régulable.

De préférence, chaque locus hétérozygote ne comporte pas d'homologie de séquence entre les deux allèles (il s'agit donc d'allèles qui contiennent des gènes hétérologues ou artificiels) ou les séquences homologues entre deux allèles dans chaque hétérozygote sont orientées inversement en ce qui concerne le sens de lecture.

De préférence, les unités d'expression incluant les éléments assurant l'expression et le gène de structure des gènes hétérologues comportent un marqueur sélectionnable lorsque ledit gène hétérologue, constitue un facteur négatif pour la vitesse de croissance de la souche.

Dans un mode de réalisation particulièrement approprié, on utilise le même locus gémomique sur chaque allèle pour l'expression de deux gènes hétérologues différents lesquels forment aussi un locus diploïde hétérozygote.

Les gènes hétérologues sont placés sous le contrôle d'un promoteur inductible et de préférence fortement régulable, par exemple au moins d'un facteur 100, ce qui permet d'éliminer la contre-sélection responsable des instabilités du type (a) mentionnées ci-dessus.

L'emploi de souches diploïdes totalement isogéniques, à l'exception du locus qui porte les gènes hétérologues et du locus sexuel (illustré ci-après par la construction des souches PES1-34 et PES1-53) permet une réduction des instabilités du type (c).

L'absence dans les diploïdes de copies sauvages (ou fonctionnellement équivalentes) aux loci hétérozygotes, chaque locus hétérozygote pouvant être constitué de gènes hétérologues (ou artificiels comme, par exemple, la cassette GAL10-CYC1-Yred-tPGK) qui répondent au critère soit d'un gène hétérologue et d'une délétion (contenant éventuellement un marqueur), permet de réduire les instabilités du type (b) mentionnées ci-dessus, tout en éliminant l'avantage sélectif d'une conversion.

L'absence dans chaque locus hétérozygote d'homologie de séquence entre les deux allèles qui contiennent des gènes hétérologues ou artificiels, ou, à défaut comme dans le cas de PES1-34 et PES1-53, l'utilisation d'une orientation inversée des séquences homologues éventuelles permet la réduction des instabilités des types (b) ou (c) mentionnées ci-dessus.

La présence d'un marqueur sélectionnable est appropriée dans toutes les unités d'expression hétérologue qui n'induisent pas de phénotype aisément identifiable, ou au minimum, dans tout allèle qui porte une fonction hétérologue constituant un facteur négatif pour la vitesse de croissance dans les conditions normales d'utilisation de la souche (le marqueur est inutile dans le cas où l'effet sur la croissance est positif, comme par exemple pour le gène modifié de la réductase).

Dans un mode de réalisation approprié la souche de levure selon l'invention est caractérisée en ce que les gènes hétérologues intégrés codent pour des facteurs constitutifs d'une chaîne de bio-conversion multi-étape ou constituant une fonction complexe.

On peut citer en particulier une souche dans laquelle sont intégrés de manière stable un gène de NADPH-Cytochrome P450-réductase de levure ou hétérologue et un gène de cytochrome b5 hétérologue.

De préférence le gène de la NADPH-cytochrome-P450 réductase est intégré dans le locus de la NADPH-cytochrome P450 réductase endogène.

On peut citer également plus particulièrement une souche de levure qui co-exprime l'époxyde hydrolase humaine et la NADPH-cytochrome P450-réductase, dont les gènes correspondants sont intégrés dans le génome de la souche de levure.

On utilisera selon l'invention plus particulièrement des souches de Saccharomyces cerevisiae.

Les souches selon l'invention sont plus particulièrement utiles dans un procédé d'expression chez la levure des facteurs constituants d'une fonction complexe notamment d'une chaîne de bioconversion multi-étape, dont l'activité principale est liée à un premier facteur hétérologue donné caractérisé en ce qu'on transforme une souche de levure selon l'invention. ladite souche co-exprimant les autres facteurs hétérologues contribuants à la fonction complexe et dans le génome chromosomique de laquelle les gènes correspondants aux dits autres facteurs sont intégrés, ladite souche étant transformée avec un plasmide portant une cassette d'expression dudit premier gène hétérologue.

En particulier, la présente invention a pour objet un procédé d'expression chez la levure de l'activité monoxygénase de cytochrome P450 héterologue caractérisé en ce qu'on transforme une souche de levure selon l'invention co-exprimant de la NADPH-cytochrome-P450 réductase et du cytochrome b5 dans le génome de laquelle sont intégrés les gènes de la NADPH-cytochrome-P450 réductase et du cytochrome b5, à l'aide d'un plasmide portant une cassette d'expression du gène du cytochrome P450 hétérologue.

De préférence le cytochrome b5 sera de la même espèce hétérologue que le cytochrome P450.

Dans ce procédé, on utilisera de préférence du cytochrome b5 humain comme stabilisateur spécifique des cytochromes P450 hétérologues dans des conditions où un haut niveau de réductase est exprimé et comme activateur spécifique de certains P450.

Outre une méthode originale et générale pour la co-intégration stable dans le génome de la levure de plusieurs gènes hetérologues, la présente invention a pour objet son application à la construction d'un ensemble PESI de souches (P450 Expression Strains série 1) qui sont adaptées plus particulièrement à l'expression de l'activité de cytochromes P 450 hétérologues. L'ensemble PESI comprend six souches haploïdes de base (pouvant être utilisées seules dans le cas des souches PES1-3, PES1-3U et PES1-4) et de trois souches diploïdes (PES1-34, PES1-31, PES1-42) formées par la combinaison des souches haploïdes de base. Chaque élément de cet ensemble peut être facilement transformé par des plasmides qui portent des cassettes d'expression pour des P450 hétérologues a priori quelconques. L'ensemble des souches (PES1-3, PES1-3D, PES1-3U, PES1-4, PES1-34, PES1-31, PES1-42) offre une large variété de contextes quant aux taux d'expression des enzymes rédox associés au fonctionnement des P450 hétérologues. Cet ensemble permet de déterminer puis d'exploiter rapidement le contexte optimal pour l'expression de chaque P450 spécifique. Les possibilités d'extension de ce concept à la co-expression d'autres activités sont présentées et illustrées par la construction d'une souche (PES1-53) qui exprime aussi l'époxyde hydrolase microsomale humaine tout en surexprimant la réductase. Les souches de levure, qui sont construites selon les méthodes présentées et qui coexpriment des cytochromes P450 hétérologues, d'origine a priori quelconque, et leur environnement enzymatique spécifique, trouvent leur application à la construction de systèmes d'évaluation et de simulation du métabolisme des médicaments et d'autres molécules xénobiotiques, à l'élaboration de systèmes de bioconversions multi-étapes, de détoxication ou de dépollution.

Les souches construites sont génétiquement stables en l'absence de toute sélection. Leurs principales caractéristiques sont :
- les souches de cet ensemble sont totalement isogéniques entre elles, à l'exception des loci spécifiquement modifiés, notamment du locus sexuel ;
- une NADPH-cytochrome P450-réductase et un cytochrome b5 sont produits dans chaque cellule, à un niveau homogène et indépendant du niveau et de la nature du cytochrome P450 hétérologue éventuellement exprimé ;
- le niveau total de la réductase et du b5 sont ajustables par la modification de la composition du milieu de culture, et ce indépendamment du niveau du P450 hétérologue éventuellement exprimé ;
- le niveau total d'expression maximal de la réductase et du b5 peuvent être au moins égaux (en terme molaire et à induction maximale) à celui du P450 hétérologue produit ;
- les niveaux minimals de la réductase et du b5 hétérologue qui peuvent être produits (en condition de non-induction) sont au moins de 10 fois inférieurs au niveau maximal de la réductase et du b5 précédemment définis ;
- la réductase peut être surproduite indépendamment du cytochrome b5 ;
- ces souches portent les marqueurs de sélection nécessaires à l'utilisation de plasmides courants, et sont, indépendamment des propriétés précédentes, de "bons hôtes" pour l'expression de cytochromes P450 hétérologues.

L'invention est également constituée de l'ensemble (PESI) des souches de levures haploïdes et diploïdes suivant :
Les éléments haploïdes :
- les éléments PES1-1 et PES1-2 ne sont pas originaux par eux-mêmes, puisqu'il s'agit respectivement des formes haploïdes, de type sexuel a et alpha, de la souche W303.1B de la levure S. cerevisiae.

L'élément PES1-3 est original ; il est construit à partir de PES1-2 de la manière suivante :
1 - les séquences génomiques de la levure PES1-2 comprises entre le premier site Kpnl situé en amont de la phase codante endogène de la NADPH-cytochrome P450 réductase et le premier codon de cette même phase codante, sont enlevées.
2 - les séquences enlevées sont remplacées à partir du Kpnl (demi-site) et dans l'ordre suivant par : le gène encodant l'orotidine-5-phosphate décarboxylase (de S. cerevisiae), un fragment du gène GAL10 de S. cerevisiae), un fragment du gène CYC1 de S. cerevisiae comprenant les séquences d'initiation de transcription de ce gène et quelques nucléotides de séquence synthétique.
   - L'élément PES1-4 est aussi original; il est déduit à partir de PES1-3 de la manière suivante :
1- les séquences génomiques comprises entre le demi-site Kpnl qui précède immédiatement le gène URA3 fonctionnel de PES1-3 (voir précédemment) et le premier site BspMI rencontré en 5' (relativement à l'orientation de la phase codante du gène de la réductase) de cette position (anciennement dans la partie promotrice du gène sauvage de la NADPH-cytochrome P450 réductase), sont enlevées.
2 - l'ensemble de la phase codante de la NADPH-450 réductase (partant du site BamHI) ainsi que sa partie flanquante en 3' jusqu'au premier site BspMI rencontré en 3' (même convention que précédemment). est enlevée.
3 - La partie éliminée est partiellement remplacée, dans l'ordre suivant et en partant de l'extrémité où etait situé le site BspMI rencontré en 3' et dans l'ordre suivant : le gène encodant l'orotidine-5-phosphate décarboxylase (de S. cerevisiae), un fragment du gène GAL10 de S. cerevisiae, un fragment du gène CYCI de S. cerevisiae comprenant les séquences d'initiation de transcription de ce gène suivi de quelques nucléotides de séquence synthétique et par la phase codante complète du gène du cytochrome b5 humain, suivie d'une courte séquence synthétique, suivie d'un fragment du gène de la phosphoglycérate-kinase de levure et comprenant des séquences 3'-flanquante de ce gène.
4 - Le signe sexuel de la souche est changé de alpha en a. les éléments diploïdes :
   La souche PSE1-34 est constituée par le diploïde des souches haploïdes PSE1-3 et PSE1-4.
   La souche PSE1-31 est constituée par le diploïde des souches haploïdes PSE1-3 et PSE1-1.
   La souche PSE1-42 est constituée par le diploïde des souches haploïdes PSE1-4 et PSE1-2.
   La souche PSE1-12 est constituée par le diploïde des souches haploïdes PSE1-1 et PSE1-2.

Enfin la présente invention a donc pour objet l'ensemble de souches de levures conformes permettant l'expression de cytochromes P450 hétérologues, chaque élément de cet ensemble pouvant être transformé par des plasmides portant une cassette d'expression du cytochrome P450 hétérologue conformément au procédé de l'invention, les différentes souches de cet ensemble ayant intégrées dans le génome de leurs chromosones les gènes de la NADPH-cytochrome P450-réductase et du cytochrome b5, gènes dont les niveaux d'expression respectifs sont variables entre les différentes souches de sorte que cet ensemble permette de déterminer la souche optimale pour l'expression de chaque cytochrome P450 spécifique.

D'autres avantages et caractéristiques de la présente invention apparaîtront à la lumière de la description detaillée qui va suivre.

La figure 1 représente la carte de restrictions de la l'ADN génomique de levure contenant le gène de la NADPH cytochrome P450-réductase de levure.

La figure 2 représente la construction du plasmide PGPIPC à partir des plasmides PGP1 et PYred-L/V8.

La figure 3 représente la construction du plasmide PGPIDT5, à partir des plasmides PGPIP et PFL39.

La figure 4 représente la construction du plasmide PYEHB1 à partir des plasmides PG1P, PYeINT1-1 et PHb5/V8.

La figure 5 représente le diagramme de construction des différentes souches à partir d'une unique souche de départ.

La figure 6 représente la linéarisation du plasmide PGPI-PC par une digestion exhaustive à l'aide de l'enzyme Eco RI et l'intégration par recombinaison homologue du fragment résultant dans le génome. La figure 7 représente l'introduction au locus endogène de la NADPH cytochrome P450-réductase de la souche PES1-1 du marqueur TRPI pour donner la souche PES1-3D.

La figure 8 représente l'introduction au locus endogène de la NADPH cytochrome P450-réductase de PES1-1 d'un gène artificiel constitué d'un promoteur hybride GAL10-CYC1, de la phase codante du cytochrome b5 humain, et de la partie terminatrice du gène PGK pour donner la souche PES1-4.

La figure 9 représente la substitution par une cassette d'expression pour l'époxide hydrolase humaine au locus de la réductase de la souche PES1-1 pour donner la souche PES1-5.

La figure 10 représente la structure physique du locus Yred dans les différentes souches.

La figure 11 représente les résultats des dosages biochimiques pour le niveau d'expression de la NADPH cytochrome P450-réductase et du cytochrome b5 dans les différentes souches.

La figure 12 représente un diagramme montrant les effets des niveaux d'expression de cytochromes b5 et réductases sur la stabilité du cytochrome humain P450 1A1.

La figure 13 représente une séparation en HPLC des métabolites du benzo(a)pyrène formé lors de l'incubation de ce polluant avec les souches PES1-2, PES1-3, et PES1-53 ou transformé pour exprimer le cytochrome P450 1A1 murin.

Le tableau 1 résume les génotypes significatifs des souches construites.

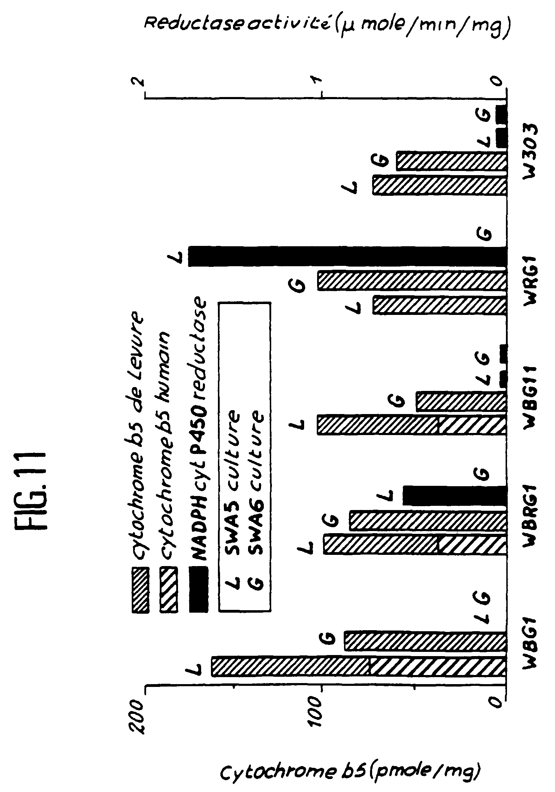

Le tableau 2 reproduit les résulats représentés dans le graphique de la figure 11.

Le tableau 3 illustre l'intérêt des souches construites pour l'expression de l'activité de cytochrome P450 hétérologue.

La méthode de construction de souches selon l'invention relève de techniques connues de l'homme de l'art et sont explicitées dans la description détaillée ci-après, en particulier le "shift" sexuel d'un haploïde par le plasmide ho, suivi de croisements des haploïdes à locus sexuel différent, pour obtenir des souches diploïdes isogéniques à l'exception du locus sexuel.
- La souche de levure de base (W303.1B) est une souche de laboratoire standard très largement diffusée. Son utilisation pour des expressions a fait l'objet de nombreuses publications.
- Les séquences de l'ensemble des gènes et fragments de gènes utilisés comme éléments de base dans les constructions, sont publiées et librement accessibles dans les banques de données. Les fragments d'ADN correspondants ont été soit reclonés ab initio au laboratoire par des approches classiques, soit recloné par PCR (Polymerase Chain Reaction) à partir des données de séquences du domaine public.
- Les techniques utilisées pour les constructions de biologie moléculaire sont classiques.

### 1. Construction des plasmides

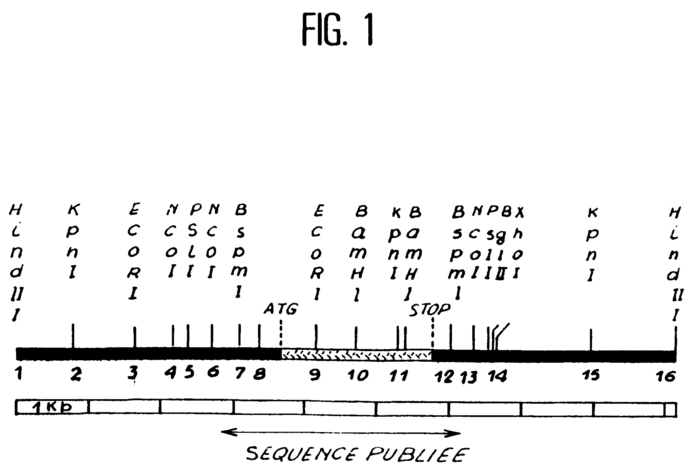

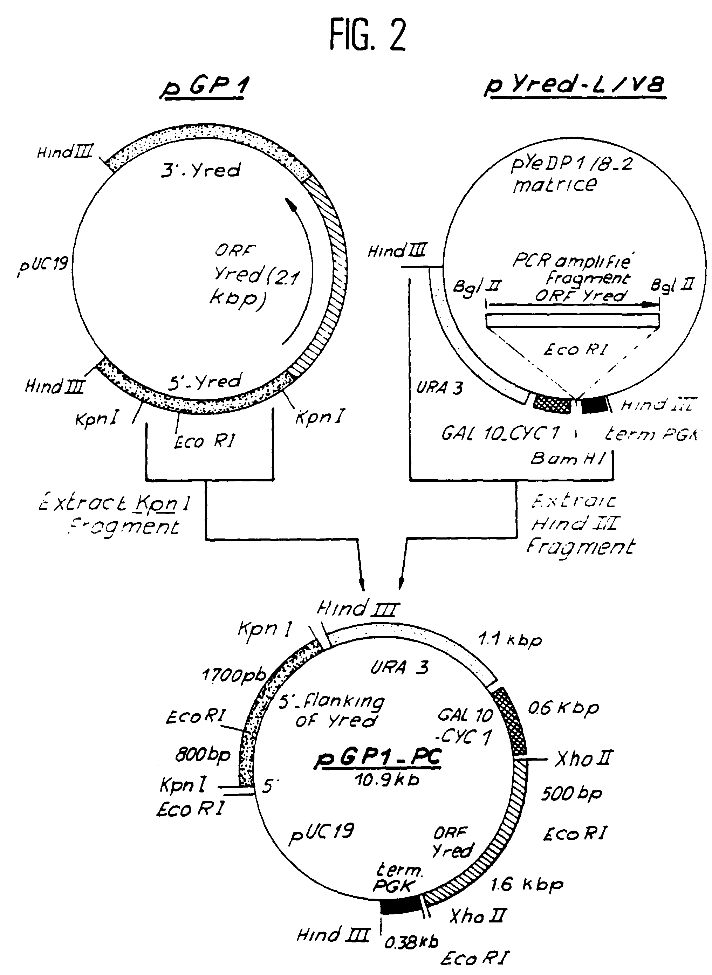

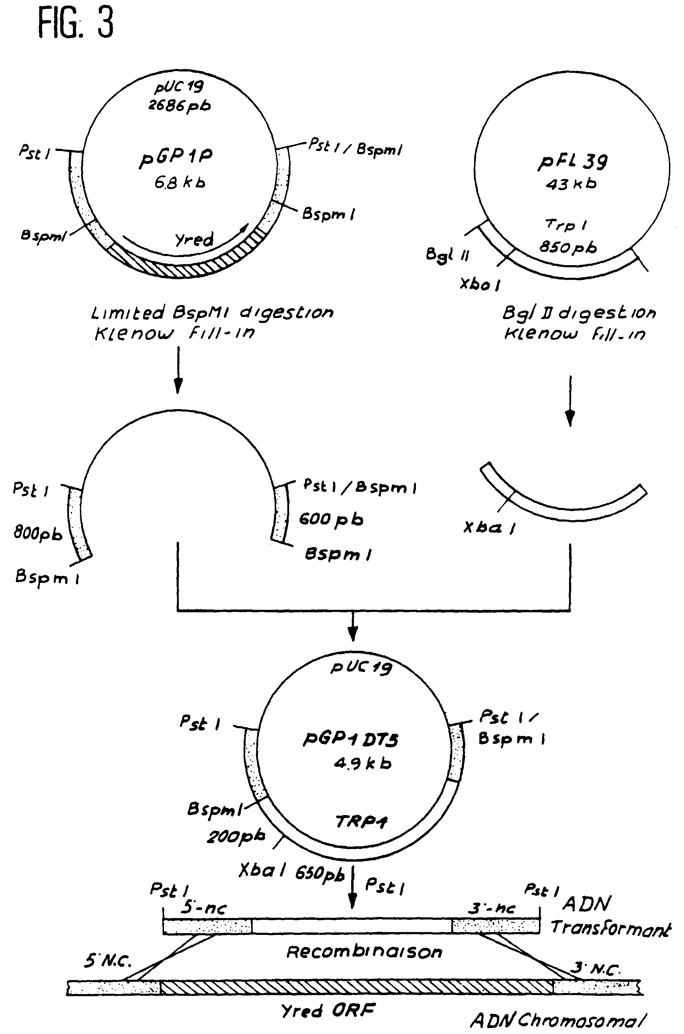

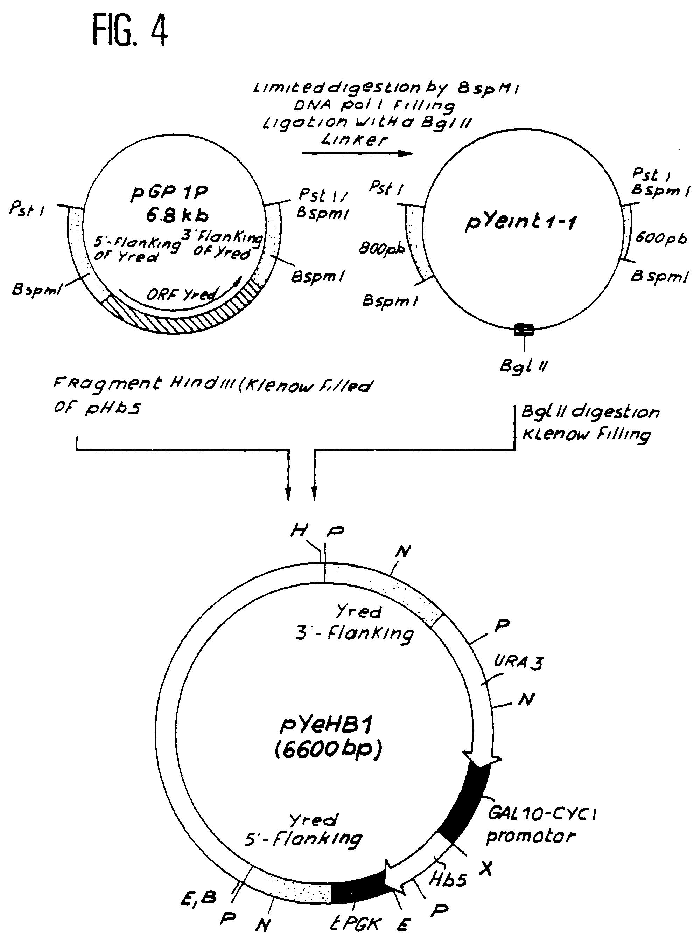

1.1. Gènes et séquences d'ADN utilisés
   - cytochrome b5 humain : la phase codante a été reclonée par PCR à partir de la séquence publiée et d'ADNc de foie humain (Yoo, M., & Steggles, A.W. (1988) Biochem. Biophys. Res. Commun. 156, 576-580 ; Urban, P., Cullin, C. & Pompon, D. (1990) Biochimie 72, 463-472).
   - NADPH-cytochrome P450 réductase : la phase codante du gène de levure a été reclonée par PCR à partir de l'ADN génomique de la souche PES1-2 et de la séquence publiée (Yabusaki, Y., Murakami, H. & Ohkawa, H. (1988) J. Biochem. (Tokyo) 103, 1004-1010 ; Urban, 1990). Les parties 5'- et 3'- flanquantes ont été obtenues à partir d'un clone isolé au laboratoire dans une banque de fragment génomique Hind III de PES1-2.
   - Epoxyde hydrolase humaine : la phase codante a été clonée par PCR à partir d'ADNc, de foie humain et de primers déduits de la séquence publiée (Skoda, R.C., Demierre, A., McBride, O.W., Gonzalez, F.J., & Meyers, U.A. (1988) J. Biol. Chem. 263, 1549-1554).
   - Marqueur URA3 et promoteur GAL10-CYC1 : les séquences ont été extraites du plasmide pLGSD5 (Guarente, L., Yocum, R.R. & Gifford, P. (1982) Proc. Natl. Acad. Sci. USA 79, 7410-7414.
   - Terminateur du gène PGK : les séquences ont été reclonées à partir des séquences publiées (Mellor, J., Dobson, M.J., Roberts, N.A., Tulte, M.F., Emtag, J.S., White, S., Lowe, P.A., Patel, T., Kingsman, A.J. & Kingsman, S.M. (1982) Gene 24, 1-14) et de PES1-2.
   - Marqueur TRPI: la séquence est extraite du plasmide pFL39 (Bonne au, N., Ozier-Kalogeropoulos, O. Li, G., Labouesse, M., Minvielle-Sebastia, L. & Lacroute, F. (1991) Yeast, sous presse).
1.2. Plasmide pGP1-PC
   Le fragment HindIII d'environ 9 kbp de l'ADN génomique de levure, contenant le gène de la NADPH-cytochrome P450 réductase de levure (Yred), a été cloné dans pUC19 pour donner pGP1. La carte de restriction approximative de l'insert est donnée sur la figure 1 en annexe. Le fragment Kpn1 e 2,5 kbp, dont l'extrémité 3' est située 333 bp en 5' de la phase codante du gène Yred (voir figure 1), est dans un premier temps cloné au site Kpn1 du vecteur pUC19 dans l'orientation qui amène le site BspMI, inclus dans le fragment, à proximité des séquences 5'-non codantes de Yred. Le vecteur résultant pGPI-PC contient environ 11 kbp (figure 2).
1.3 Plasmide pGP1-DT5
   Le fragment Pst1 de 4,2 kbp de pGP1 qui contient le gène Yred est sous-cloné dans le site Pst1 de pUC19 pour donner pGPIP. Le plasmide pGPIP est alors soumis à une digestion partielle par BspMl et le fragment de 4 kbp, qui en résulte et qui contient pUC19 et les séquences 5'- et 3'- flanquantes de Yred, est utilisé comme vecteur pour cloner le fragment de 850 bp contenant le gène TRPI de levure (obtenu par une digestion totale du plasmide pFL39 (Bonneau, 1991) par BgIII suivie d'un traitement par la DNA polymérase). L'orientation du fragment cloné est telle que le site Xbal du gène TRPI est situé à 200 bp du site BspMI de la partie 5'-flanquante du gène Yred (voir figure 3).
1.4- Plasmide pYeHB1
   Le plasmide pGP1P est soumis à une digestion partielle par BspMI pour éliminer le fragment de 2875 bp portant la phase codante de Yred. Ce fragment est remplacé par un oligonucléotide de 10 bp portant un site BgIII donnant pYeint 1-1 (figure 4). Le fragment HindIII de 2,5 kbp de Hb5/V8 (Urban, 1990) qui contient le gène URA3, le promoteur GAL10-CYC1, la phase codante du cytochrome b5 humain et la partie terminatrice du gène PGK est cloné (après avoir été rendu bout-franc) dans le vecteur PYeint1-1 ouvert au site BgIII (site bouché par traitement à la DNA pol.1). L'orientation sélectionnée est telle que le vecteur résultant contienne le gène URA3 de manière contiguë à la partie 3'-non codante du gène Yred (fig 4).
1.5- Plasmide pYeHEH
   La phase codante de l'époxyde hydrolase microsomale humaine est amplifiée par PCR (de manière analogue à la méthode utilisée pour cloner le cytochrome b5) en utilisant deux primers complémentaires sur 24 nucléotides aux extrémités 3' de la séquence publiée. L'extrémité 5' de chaque primer inclus un site BamHI non homologue. Le fragment BamHI qui en résulte est alors cloné au site BamHI du vecteur pYeDP 1/8-2 (Cullin, C., & Pompon, D. (1988) Gene 65, 203-217) dans la bonne orientation. Le fragment HindIII qui contient la cassette d'expression est alors extrait du plasmide obtenu et les extrémités 3'-rentrantes sont remplies par un traitement à la Klenow pour permettre le clonage dans le vecteur PYeINT1-1 ouvert au site BgIII puis les extrémités sont remplies de même à l'aide de la Klenow. L'orientation sélectionnée est telle que le vecteur qui en résulte contienne le marqueur URA3 de manière contiguë à la partie 3'-non codante du gène Yred original. Le plasmide pYeHEH qui en résulte est semblable à pYeHB1 excepté la présence de la phase codante de l'époxyde hydrolase humaine en lieu et place de celle du cytochrome b5 humain.

### 2. Construction des souches de levure

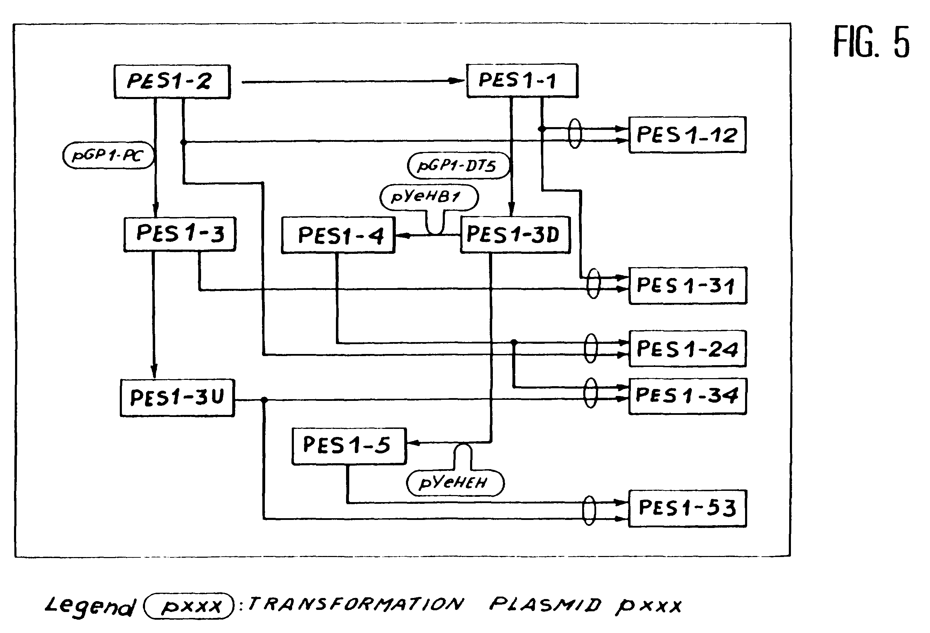

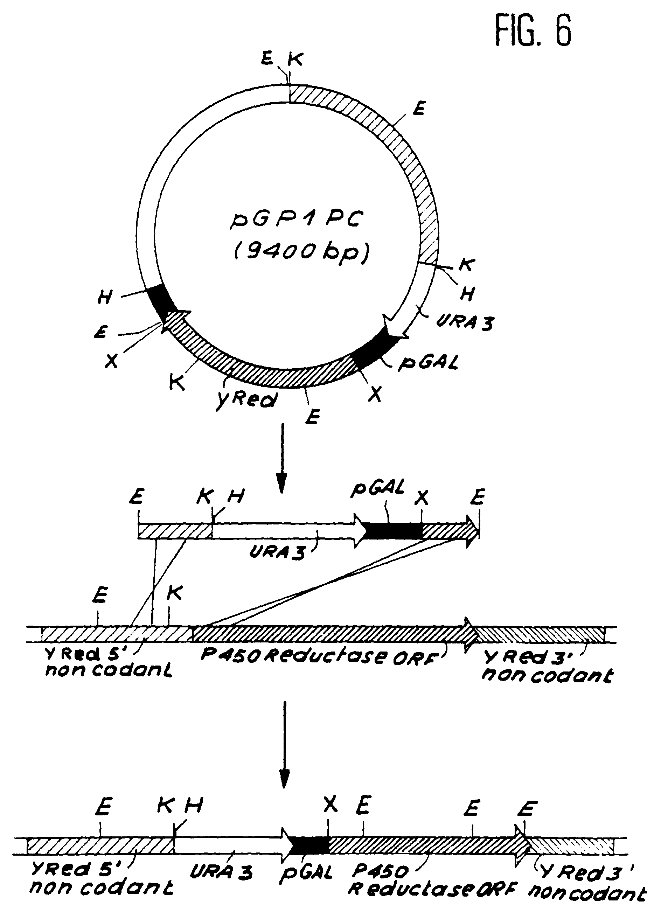

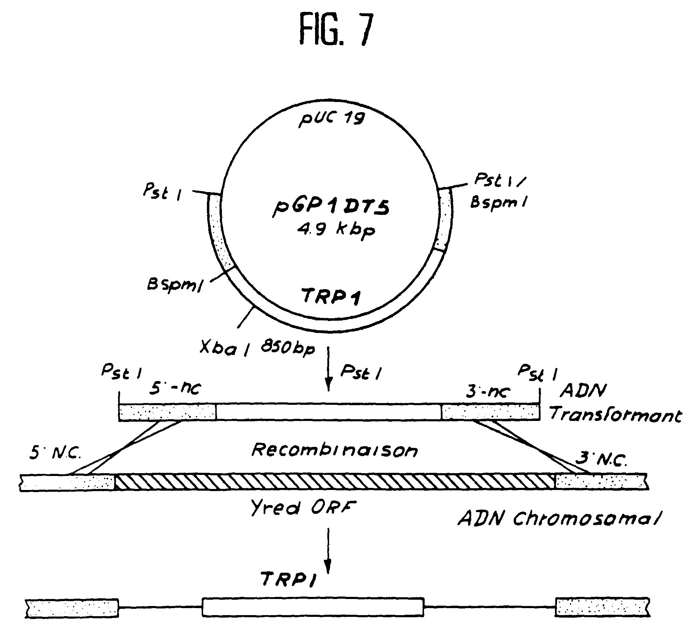

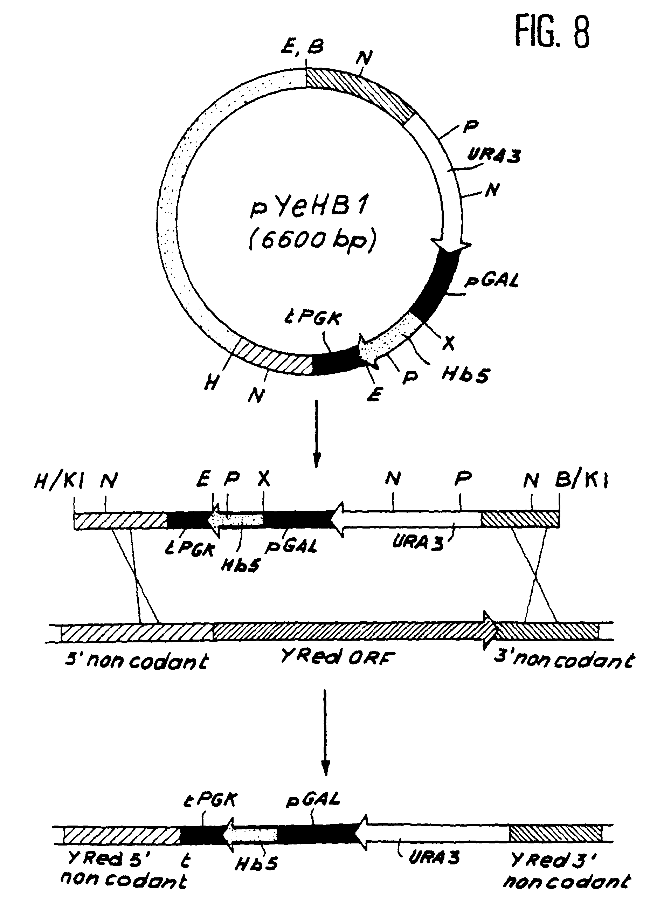

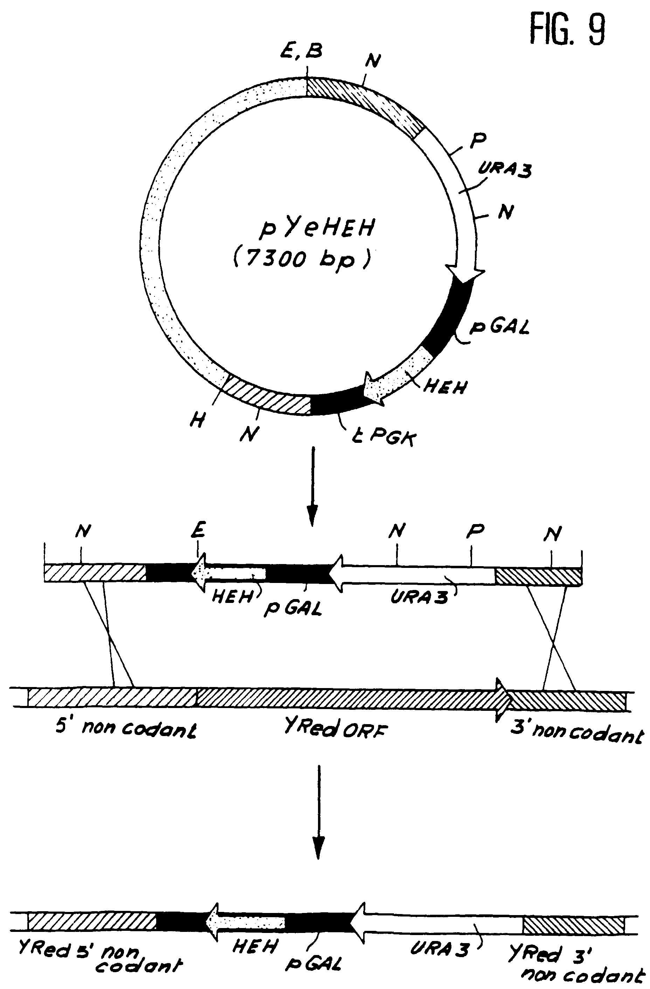

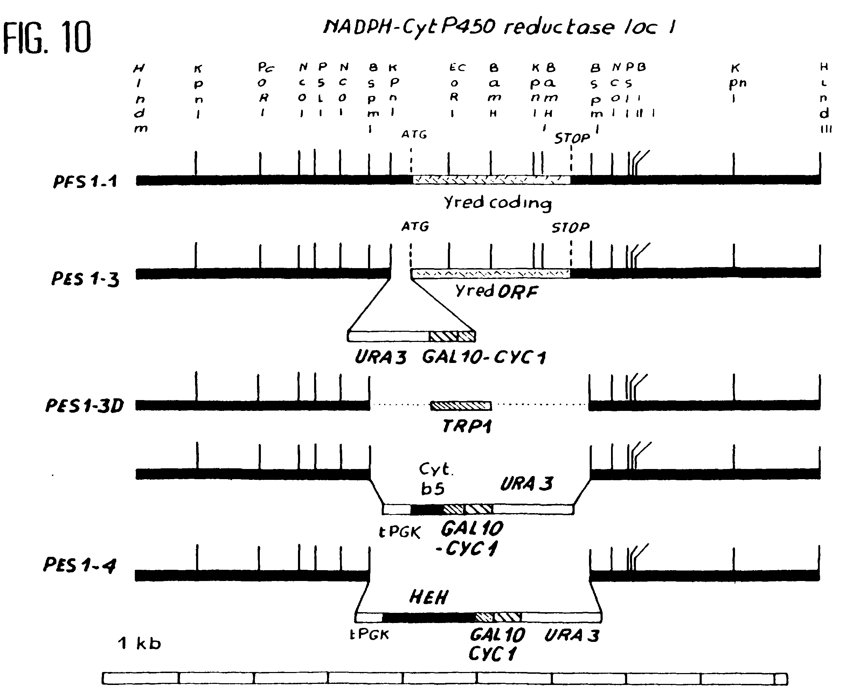

2.1 Généralités
   Toutes les souches de levure ont été construites (voir Figure 5) à partir d'une souche de départ unique connue de l'homme de l'art sous le nom de W303.1B. Cette souche est haploïde (mating type alpha) et porte comme mutations connues ura3, Ade2, His 1, Trp1, Leu2 ; elle respire normalement et est capable d'utiliser le galactose comme source de carbone. Par un souci d'homogénéité cette souche est désignée par la suite sous le nom de PES1-2. La structure du locus Yred des différentes souches est résumée sous forme graphique dans la figure 10.
2.2 Souche PES1-1
   Cette souche haploïde (mating type a) a été construite à partir de la souche PES1-2 par l'échange sexuel grâce à la technique du plasmide Ho appliquée selon la méthode publiée (Methods in Enzymology, vol.194, pp132-146). La souche PES1-1 est donc totalement isogénique (à l'exception du locus sexuel) à la souche PES1-2.
2.3 Souche PES1-12
   Cette souche est le diploïde résultant simplement du croisement entre les souches PES1-1 et PES1-2. Le caractère diploïde est vérifié par la faculté de sporuler en milieu acétate. Du fait de la construction, ce diploïde est homozygote pour l'ensemble de ses gènes (locus sexuel excepté).
2.4 Souche PES1-3 et PES1-3U
   Cette souche haploïde (mating type alpha) est totalement isogénique à PES1-2, sauf le locus endogène de la NADPH-cytochrome P450 réductase qui est modifié de façon à placer la phase codante naturelle de la réductase sous le contrôle du promoteur inductible GAL10-CYC1 par la procédure suivante :
   1- la souche PES1-2 est transformée (en utilisant la méthode standard au chlorure de lithium) par le plasmide pGP1-PC doublement linéarisé par une digestion exhaustive à l'aide de l'enzyme Eco RI (voir figure 6) et les transformants URA+ sont sélectionnés.
   2- Les transformants ainsi sélectionnés sont triés sur la base d'une croissance lente par rapport à PES1-2 en milieu complet glucosé (YPGA) et d'une croissance identique à PES1-2 en milieu complet galactosé(YPGaIA).
   3- un des clones qui répondent à ces critères est choisi et stocké sous le nom de PES1-3.
   4- des mutants spontanés de phénotype ura3 de PES1-3 sont sélectionnés par une réplique faite sur un milieu contenant de l'uracile et de l'acide 5-fluoro-orotique. Un mutant URA⁻ stable (taux de réversion inférieur à 10-7), indistinguable par tout autre critère de PES1-3, est stocké sous le nom de PES1-3U.
   5- le génotype au locus de la NADPH-cytochrome P450 réductase et le phénotype relatifs sont ensuite testés (voir 3.1).
2.5- Souche PES1-3D
   Cette souche haploïde est totalement isogénique à PES1-1 sauf pour le locus endogène de la NADPH-cytochrome P450 réductase. Ce locus a été modifié de façon à éliminer l'ensemble de la phase codante ainsi qu'une partie des séquences 5'- et 3'-flanquantes, un marqueur TRPI est alors introduit au niveau de la délétion (voir figure 7).
   1- la souche diploïde PES1-12 est transformée (en utilisant la méthode standard au chlorure de lithium) par le plasmide pGP1-DT5 doublement linéarisé par une digestion exhaustive à l'aide de l'enzyme Pst1, et les transformants TRP+ sont sélectionnés.
   2- les cellules diploïdes de quelques clones ainsi obtenus, sont mises à sporuler sur milieu acétate. Les tétrades résultantes sont disséquées.
      Les clones haploïdes TRP+ qui présentent une croissance lente par rapport à PES1-1 sur milieu complet glucosé YPGA, sont sélectionnés puis testés par croisement pour déterminer leur signe sexuel et leur capacité à croître sur un milieu galactosé (YPGaIA) et sur un milieu glycérolé (N3).
   3- un des clones de signe sexuel "a", qui répond à ces critères, est choisi et stocké sous le nom de PES1-3D.
   4- le génotype au locus de la NADPH-cytochrome P450 réductase et le phénotype relatifs sont testés (voir 3.1).
2.6- Souche PES1-4
   Cette souche haploïde (mating type alpha) est totalement isogénique à PES1-1, à l'exception du locus endogène de la NADPH-cytochrome P450 réductase. Ce locus a été modifé de façon à éliminer l'ensemble de la phase codante de la réductase (ainsi qu'une partie des séquences 5'- et 3'- flanquantes). Un gène artificiel constitué du promoteur hybride GAL10-CYC1, de la phase codante du cytochrome b5 humain, et de la partie terminatrice du gène PGK est alors introduit à l'emplacement libéré par la procédure suivante (voir figure 8).
   La souche haploïde PES1-3D est transformée (en utilisant la méthode au chlorure de lithium) par le plasmide pYe-HB1 doublement linéarisé par une digestion exhaustive à l'aide des enzymes BamHI et HindIII, puis les transformants URA+ sont sélectionnés.
   Les clones haploïdes URA⁺ ayant un phénotype TRP⁻, une croissance lente (par rapport à PES1-1) sur milieu YPGA ou YPGa1A et poussant sur milieu glycérolé (N3) sont sélectionné.
   Le génotype relatif au locus de la NADPH-cytochrome P450 réductase et le phénotype au niveau du cytochrome b5 sont testés (voir 3.1.).
2.7- Souche PES1-31
   Cette souche est le diploïde formé à partir des souches PES1-3 (URA3) et PES1-1 (Ura3). Après le croisement, la sélection est basée sur la recherche de diploïdes URA+. Cette souche, de même que PES1-34 et PES1-42, n'est hétérozygote que pour le locus de la NADPH-cytochrome P450 réductase et pour le locus sexuel. Une des copies du gène de la réductase est sauvage, l'autre est sous le contrôle du promoteur GAL10-CYC1 (elle inclut le marqueur URA3).
2.8- Souche PES1-34
   Cette souche est le diploïde formé à partir des souches PES1-3U (Ura3) et PE1-4 (URA3). Après le croisement, la sélection est basée sur la recherche de diploïdes URA+. Une seule copie modifiée du gène de réductase est présente sous le contrôle du promoteur CAL10-CYC1 (cette souche inclut un marqueur Ura3 inactif), dans l'autre locus la phase codante de la réductase est délétée et remplacée par la cassette d'expression pour le cytochrome b5 (qui inclut un marqueur URA3 fonctionnel).
2.9- Souche PES1-42
   Cette souche est le diploïde formé à partir des souches PES1-4 (URA3) et PES1-2 Ura3). La sélection après croisement est basée sur la recherche de diploïdes URA+. Cette souche contient une seule copie sauvage fonctionnelle du gène de la P450 réductase, le second locus de la réductase est occupé par la cassette d'expression pour le cytochrome b5 (qui inclut un marqueur URA3 fonctionnel).
2.10- Souche PES1-5
   Cette souche est isogénique à la souche PES1-1 à l'exception du locus de la réductase qui a été substitué par une cassette pour l'époxyde hydrolase humaine de la façon suivante (voir figure 9) :
   1- La souche PES1-1 est transformée par le vecteur pYeHEH linéarisé par une double digestion exhaustive par les enzymes HindIII et SalI, les clones URA3 sont sélectionnés.
   2- Parmi les transformants, les clones qui ont une vitesse de croissance similaire à celle de PES1-3D sur un milieu complet glucosé (YPGA) et qui poussent sur un milieu glycérolé (N3), sont sélectionnés.
   3- Un clone exprimant l'époxyde hydrolase humaine est sélectionné et stocké sous le nom de PES1-5.
   4- Le génotype relatif au locus de la NADPH-cytochrome P450 réductase et la phénotype relatif au niveau de l'époxyde hydrolase sont testés (voir 3.1).
2.11 Souche PES1-53
   Cette souche est le diploïde formé à partir des souches PES1-5 et PES1-3U. La sélection après le croisement est basée sur la recherche d'un diploïde URA+. Cette souche diploïde contient :
   - sur l'un des deux loci originaux de la réductase, la phase codante Yred sous promoteur GAL10-CYC1 (ce locus comporte un marqueur Ura3 inactif).
   - l'autre locus original de la réductase est délété et remplacé par une cassette d'expression pour l'époxyde hydrolase humaine, il contient aussi un marqueur URA3 actif.

### 3. Caractérisation génétique des souches construites

### 3.1- Nature des génotypes

Les génotypes significatifs des souches construites sont résumés dans le tableau 1. La structure physique du locus Yred dans les différentes souches est donné dans la figure 10, elle a été contrôlée comme suit :
- PES1-1, PES1-2, PES1-12 : loci Yred sauvages.
- PES1-3 et PES1-3U : (al) amplification par PCR de l'ORF de Yred (2,1 kbp) en utilisant deux primers situés respectivement aux extrémités 5' et 3' des brins codants et non-codants de la phase codante. (bl) amplification par PCR d'une bande de 2,2 kbp en utilisant un premier primer situé à l'extrémité 3' du promoteur GAL10-CYC1 et un second situé à l'extrémité 5' du brin non codant de la phase codante. (c1) Southern blot de l'ADN génomique après une digestion par ECORI, et revélation avec une sonde fabriquée à partir de l'ORF de Yred.
- PES1-3D : (a2) absence d'amplification des bandes de 2.1-2.2. kbp des tests "a1 et b1". (b2) test identique à "c1" en utilisant une sonde TRP1.
- PSE1-4 : (a3) amplification par PCR de l'ORF du cytochrome b5 (405 bp) en utilisant deux primers situés respectivement aux extremités 5' et 3' des brins codants et non-codants de la phase codante.

Absence d'amplification comme dans le test "a2". (b3) amplification par PCR d'une bande de 0.40 kbp en utilisant un premier primer situé à l'extrémité 3' du promoteur GAL10-CYC1 et un second situé à l'extrémité 5' du brin non-codant de la phase codante du b5. (c3) Southern blot de l'ADN génomique après une digestion par EcoRI puis révélation par une sonde fabriquée à partir de la phase codante du b5 humain.
- PES1-31 : (a4) tests "b1 et c1", contrôle de l'état diploïde par sporulation.
- PES1-34 : (a5) tests "a1,b1,b3,c1,c3", contrôle de l'état diploïde par sporulation.
- PES1-42 : (a6) tests "a1,b3,c3", contrôle de l'état diploïde par sporulation.
- PES1-5 et PES1-53 : mêmes contrôles que pour PES1-4 et PES1-34 respectivement, mais en utilisant la séquence de l'époxyde hydrolase au lieu de celle du cytochrome b5.

Les auxotrophies de l'ensemble de souches sont déterminées par une réplique sur des boîtes de milieu minimum partiellement complementées.

### 4. Dosages enzymatiques

4.1- cultures et préparation de fractions microsomales
   Les différentes souches sont cultivées à 28°C sous une agitation modérée (agitateur orbital à 100 rpm) dans un milieu semi-synthétique composé comme suit.
   - milieu SWA6 : D-glucose 20g/l (w/v), yeast nitrogen base (Difco) 0,7% (w/v), Hydrolysat acide de caséine 0,1% (w/v), adénine 40 mg/l, tryptophane 20 mg/l.
   - milieu SWA5 : D-galactose 20g/l (w/v), yeast nitrogen base (Difco) 0,7% (w/v), Hydrolysat acide de caséine 0,1% (w/v), adénine 40 mg/l, tryptophane 20mg/l.
   - milieu SW6 : D-glucose 20g/l (w/v), yeast nitrogen base (Difco) 0,7% (w/v), Hydrolysat acide de caséine 0,1% (w/v), tryptophane 20mg/l.
   - milieu SW5 : D-galactose 20g/l (w/v), yeast nitrogen base (Difco) 0,7% (v/w), Hydrolysat acide de caséine 0,1% (w/v), tryptophane 20mg/l.

   Les cultures (250ml) sont arrêtées lorsque la densité cellulaire atteint 2,5 X 10⁷ cellules par ml. Les cellules sont alors collectées par une centrifugation de 5 min à 2500g. Le culot cellulaire est repris dans 35 ml de tampon 50mM Tris-HCI pH7.4, 5 mM EDTA, 100 mM KCl, 20 mM bêta-mercaptoethanol, puis les cellules sont incubées durant 10 min à 20°C puis recentrifugées 3 min à 10000 g. Le culot cellulaire est lavé par 35 ml de tampon 50mM Tris-HCl pH7.4, 1 mM EDTA, 0.6 M sorbitol (dit tampon de casse), puis la suspension cellulaire est centrifugée dans des tubes à bouchon à vis de 45 ml (comme précédemment). Un volume de tampon de casse égal au double du volume du culot est alors ajouté pour former une suspension très dense. Des billes de verre (du type Braun de diamètre 0.45-0.5 mm), sont aussitôt ajoutées en une quantité juste suffisante pour affleurer la surface de la suspension cellulaire. Les tubes sont ensuite secoués verticalement et vigoureusement (3 coups par seconde) durant deux minutes. 3 ml de tampon de casse sont alors ajoutés, et les tubes sont vortexés pendant 5 secondes. Le liquide présent entre les billes (mais non les billes) est transféré dans un autre tube. 3 ml de tampon de casse sont à nouveau ajoutés aux billes résiduelles, et l'opération (vortex et transfert) est recommencée. L'extraction est répétée une troisième fois. L'ensemble des extraits (environ 10ml) est centrifugé 15 min à 10000g. Le surnageant est transféré dans un autre tube et le volume ajusté à 10ml avec du tampon de casse. Trois cents microlitres d'une solution de NaCI 4M sont ajoutés puis trois millilitres et demi d'une solution aqueuse de polyéthylène glycol-4000 à 40% (w/v). Le mélange est laissé 15 min à 0°C, puis centrifugé 10 min à 10000g. Le surnageant est soigneusement éliminé et la surface du culot (qui contient la fracton microsomale) et du tube sont lavés slans resuspendre le culot par un faible volume de tampon 50mM Tris-HCI pH7.4, 1 mM EDTA, 20% (v/v) glycérol (dit tampon de reprise). Le culot est resuspendu dans un millilitre de tampon de reprise et la suspension est homogénéisée par aspiration et refoulement dans une seringue munie d'une aiguille (coudée deux fois en Z) de diamètre 0.3 mm. Les microsomes sont alors aliquotés par 200 µl et conservés congelés à -80°C.
4.2- dosage des activités
   - NADPH-cytochrome P450 réductase : l'activité est testée en mesurant la réduction du cytochrome c de coeur de cheval (Sigma type VI) à une concentration de 10 µM, en présence de NADPH (100 µg/ml), et dans un tampon Tris-HCI 50mM pH 7.4, 1 mM EDTA. L'activité est calculée en utilisant un coefficient d'absorption différentiel de 21 mM-1 à 550 nm. Une unité d'activité est définie comme la quantité d'enzyme (sous forme de suspension de microsomes) capable de réduire 1 nanomole de cytochrome c par minute à 20°C. L'activité est rapportée à la quantité totale de protéines microsomales déterminée à l'aide du test BCA de Pierce.
   - Cytochrome b5 et cytochrome P450 : La concentration de ces cytochromes est déterminée par spectrophotométrie différentielle comme décrit auparavant (Urban, 1990).
   - L'activité EROD (EthoxyRésorufine-O-Dééthylase) du cytochrome P450 1A1 est déterminée en mesurant à 22°C l'accroissement de fluorescence à 586 nm (excitation réglée à 530 nm) dans une cuve contenant du tampon Tris-HCI 50 mM pH 7.4 l mM EDTA, 0.1 mg/ml de NADPH, 1 µM de 7-éthoxyrésorufine et un aliquote (généralement : 10 µl d'une suspension de microsomes). L'activité est calculée et exprimée en picomole de résorufine formées par min après calibration de l'appareil avec de la résorufine authentique.
   - L'activité testostérone 6-bêta hydroxylase du P450 IIIA4 est mesurée en incubant durant 15 min un aliquote de la suspension de microsomes (généralement : 20 µl) avec 230 µl de tampon Tris-HCI 50 mM pH 7.4, 1 mM EDTA, 0.05 mg/ml de NADPH, 80 µM de testostérone. La réaction est arrêtée par addition de 5 µl d'acide trifluoroacetique, la phase aqueuse est extraite par 500 µl de dichlorométhane. Après l'évaporation du solvant sous un courant d'azote, le résidu sec (les stéroïdes) est repris dans 40 µl d'un mélange méthanol-eau (1:3 v/v), les métabolites formés sont séparés par HPLC (chromatographie liquide à haute pression) sur une colonne de phase réverse de C18 éluée par un gradient acétonitrile-eau. L'identification et la quantitification des métabolites sont effectuées par une comparaison de calibration avec les produits authentiques.
   - Epoxyde hydrolase : l'activité époxyde hydrolase est mesurée en incubant un aliquote de la suspension de microsomes (généralement 40 µl) dans 400 µl de tampon Tris-HCI 50mM pH 7.4, 1 mM EDTA, contenant 1mM de styrène-oxyde. La formation du diol correspondant est quantifiée par séparation en HPLC sur une colonne réverse de C18 en utilisant le diol authentique comme référence.
   - L'activité de bioconversion du benzo(a)pyrène en benzo(a)pyrène-7,8-diol est démontrée sur des fractions microsomales de la souche PES1-53 transformée par le plasmide d'expression ccP1/V60 (Cullin et Pompon, 1990) qui code le cytochrome P450 de la famille 1A1 murine. Une quantité de microsomes de levure qui correspond à 100 µg de protéines totales, est incubée durant 15 minutes avec 250 µl de tampon Tris-HCI 50 mM pH7.4, 1 mM EDTA, 15 µM en benzo(a)pyrène et contenant 0.1 mg/ml de NADPH. La réaction est arrêtée par l'addition de 10 µl d'acide trifluoroacétique. Après la saturation de la phase aqueuse avec du chlorure de sodium et l'extraction des métabolites avec du dichlorométhane, les métabolites sont séparées par HPLC en phase reverse de C18 selon des procédures standard.

   L'ensemble des souches construites constitue :
   - un outil analytique permettant d'identifier rapidement le contexte enzymatique rédox associé aux P450 optimal pour l'expression d'une activité définie d'un P450 donné.
   - un outil de production après l'identification, dans cet ensemble, de la souche la mieux adaptée aux besoins.
4.3. Les propriétés individuelles des souches de cet ensemble sont :
   . Souche PSE1-3 :
      Le travail de génie génétique effectué sur cette souche a pour conséquence de détruire la régulation naturelle du gène de la NADPH-cytochrome P450 réductase de levure et de placer la synthèse de cette protéine sous le contrôle transcriptionel d'un promoteur hybride artificiel constitué de l'UAS (Upstream Activating Sequence) du gène de levure GAL10 et des séquences d'initiation de transcription du gène de levure CYC1.
      Cette souche exprime, lorsqu'elle est cultivee sur un milieu contenant du galactose, un niveau de NADPH-cytochrome P450 réductase qui est 20 à 30 fois supérieur au niveau présent dans la souche parentale PSE1-2.
      A l'inverse, en l'absence de galactose, le niveau de réductase présent est extrêmement faible (en fait indétectable), c'est à dire au moins 100 fois inférieur au niveau présent dans le parent PSE1-2). Des niveaux d'expression intermédiaires entre ces deux extrêmes sont possibles en utilisant, soit des durées d'induction par le galactose limitées (0 à 12 heures), soit des cultures en présence d'un mélange adapté de glucose et de galactose.
      A noter un fait très important pour des utilisations biotechnologiques : les souches PSE1-3 et PES1-31 ne contiennent pas de séquence d'ADN exogène à la levure (en particulier bactérienne). Les rares fragments de séquence synthétique introduits sont très courts (inférieurs à 10 bp) et de séquences "banales" susceptibles d'être trouvées naturellement en de nombreux exemplaires chez l'hôte.
   . Souche PSE1-4 :
      Cette souche présente deux caractétistiques :
      - elle n'exprime pas de NADPH-cytochrome P450 réductase (ni endogène, ni exogène).
      - elle exprime à la fois un niveau normal (celui d'une souche sauvage) de cytochrome b5 de levure et un niveau conditionnel (élevé en milieu de culture galactosé, très faible en milieu de culture glucosé) de cytochrome b5 humain. En conséquence, le niveau total (levure + humain) de cytochrome b5 exprimé, en présence de galactose comme seule source de carbone, est 2 à 3 fois supérieur au niveau de b5 endogène de la souche sauvage haploïde PSE1-1.
   . Souche PSE1-34 :
      Lorsqu'elle est cultivée sur un milieu de culture contenant du glucose comme source de carbone, cette souche exprime un niveau total de NADPH-cytochrome P450 réductase 2 fois inférieur à la souche PSE1-12 (la diploïde sauvage de référence), un niveau de cytochrome b5 de levure normal (niveau de la souche sauvage de référence), un niveau de cytochrome b5 humain négligeable (au moins 100 fois inférieur au niveau de cytochrome b5 de levure).
      Lorsqu'elle est cultivée sur un milieu de culture contenant du galactose come source de carbone, cette souche exprime un niveau total de NADPH-cytochrome P450 réductase environ 10 fois supérieur au niveau de la souche sauvage de référence, un niveau de cytochrome b5 de levure équivalent au niveau sauvage et un niveau de cytochrome b5 humain 2 à 3 fois supérieur au niveau de cytochrome b5 de levure.
   . Souche PSE1-31
      Cette souche exprime en milieu de culture glucosé, un niveau de cytochrome P450 réductase égal à la moitié du niveau du diploïde sauvage PSE1-21. En milieu de culture galactosé, elle exprime un niveau de NADPH-cytochrome P450 réductase similaire à celui de la souche PSE1-34. Cette souche n'exprime pas de cytochrome b5 hétérologue, mais exprime un niveau sauvage de cytochrome b5 endogène.
   . Souche PSE1-42
      Quelle que soient les conditions de culture (glucose ou galactose) cette souche exprime les mêmes niveaux de cytochromes b5 (endogènes et humain) que la souche PSE1-34 et un niveau de réductase d'environ moitié que la souche diploïde sauvage PSE1-12.
      Le tableau 2 résume les principales caractéristiques des ces souches. 4.4. Résultats des dosages biochimiques
4.4.1 Niveau d'expression de la NADPH cytochrome P450 réductase et du cytochrome b5
   Le niveau d'expression de la NADPH cytochrome P450 et des cytochromes b5 (la forme endogène de levure et la forme humaine) ont été déterminés dans des conditions standard de culture (voir 4.1) pour les souches construites. Les résultats sont présentés d'une part dans le tableau 2, d'autre part sous forme graphique dans la figure 11. Les résultats appellent les commentaires suivants :
   - l'activité réductase dans la souche PES1-3 cultivée en milieu galactosé SWA5 est accrue environ 30 fois par rapport à l'activité de la souche sauvage. Cette augmentation est de l'ordre de 10 fois dans les souches PES1-34 et PES1-31. En milieu glucosé SWA6 (milieu de répression) le niveau de réductase est par contre indétectable (au moins 100 fois inférieur au niveau de la souche sauvage).
   - l'activité du niveau d'expression du cytochrome b5 pose un problème analytique. En effet, la levure exprime de manière endogène un cytochrome b5 qui n'est pas distinguable du cytochrome b5 humain dans le test de dosage utilisé (qui est sans relation avec l'activité cherchée mais est le seul test quantitatif disponible). Le cytochrome b5 endogène, bien que présent en quantité substantielle, ne semble pas équivalent au cytochrome b5 de mammifère pour le but souhaité, qui est l'étude de l'interaction du cytochrome b5 avec les P450 humains produits dans la levure, ce qui justifie l'expression du cytochrome b5 hétérologue humain. Les contributions respectives des deux formes de cytochromes b5 dans le dosage utilisé (qui dose la somme des deux) peut néanmoins être estimée par une analyse de l'ensemble des données (voir table 2 et figure Il), en supposant une expression proportionnelle au dosage génique dans les souches diploïdes. Cette analyse ne permet par un calcul univoque de la concentration en cytochrome b5 humain du fait de l'ignorance des mécanismes de régulation du cytochrome b5 endogène en réponse à l'expression d'un cytochrome b5 exogène, mais il conduit à une fourchette de niveaux d'expression centrée en ordre de grandeur autour de 100 pmole par mg de protéines microsomales. Le point important est qu'un tel niveau est en stoechiométrie correcte (les P450 forment un complexe 1/1 en mole avec le b5) avec le niveau auquel les P450 sont produits dans la levure en utilisant les technologies actuelles. Le faible accroissement apparent (environ deux fois) du niveau de cytochrome b5 total dans les souches construites est néanmoins fonctionnellement très significatif du fait de la non équivalence des cytochromes b5 de levure et humain (voir ci-dessous).
4.4.2. Influence du niveau de réductase et de cytochrome b5 sur l'activité de cytochromes P450 hétérologues
   L'intérêt des souches construites pour l'expression de l'activité de cytochromes P450 hétérologues est illustré par des exemples décrits dans le tableau 3. Ces exemples sont donnés à titre d'illustration et ne sont pas limitatifs des applications possibles. Les données résultent de l'expérience suivante :
   - des plasmides d'expression qui contiennent les phases codantes des cytochromes P450 1A1 murin et P450 1A1 et 3A4 (sous-type NF25) humains clonées entre les sites BamHI et ECORI du vecteur d'expression de levure pYeDP60 (Cullin,1988 ; Renaud, J.P., Cullin, C., Pompon, D., Beaune, P., & Mansuy, D. (1990) Eur. J. Biochem. 194, 889-896 ; Urban,1990, Gautier, J.C. et coll. (1991) manuscrit en préparation), sont introduits dans les souches de la série PES1 (par une transformation au chlorure de lithium) et les clones qui présentent une prototrophie pour l'adénine sont sélectionnés. Les transformants sont cultivés en milieu synthétique galactosé SW5 jusqu'à ce qu'une densité de 2.2 x 10⁷ cellules par ml soit atteinte, puis les fractions microsomales sont préparées (Cullin,1988 ; Renaud,1990 ; Urban,1990 ; Gautier,1991). Les fractions microsomales sont testées pour les activités 7-éthoxyrésorufine-O-dééthylase (EROD), testostérone 6-bêta hydroxylase et benzo(a)pyrène monooxygénase comme décrit précédemment.

   Les résultats appellent les commentaires suivants :
   - la surproduction de la P450 réductase, dans PES1-3 par exemple, induit une augmentation significative (de 5 à 10 fois selon les conditions) de l'activité spécifique (en valeur de turn-over des enzymes) des cytochromes P450 de la famille 1A1 testés. Ce facteur d'augmentation atteint de 40 à 60 fois dans le cas du P450 3A4 humain. Néanmoins, cette augmentation d'activité spécifique s'accompagne, dans le cas du P450 1A1, de la destruction d'une part significative de l'enzyme probablement par des processus oxidatifs qui induisent la conversion de la forme P450 active en la forme P420 inactive.

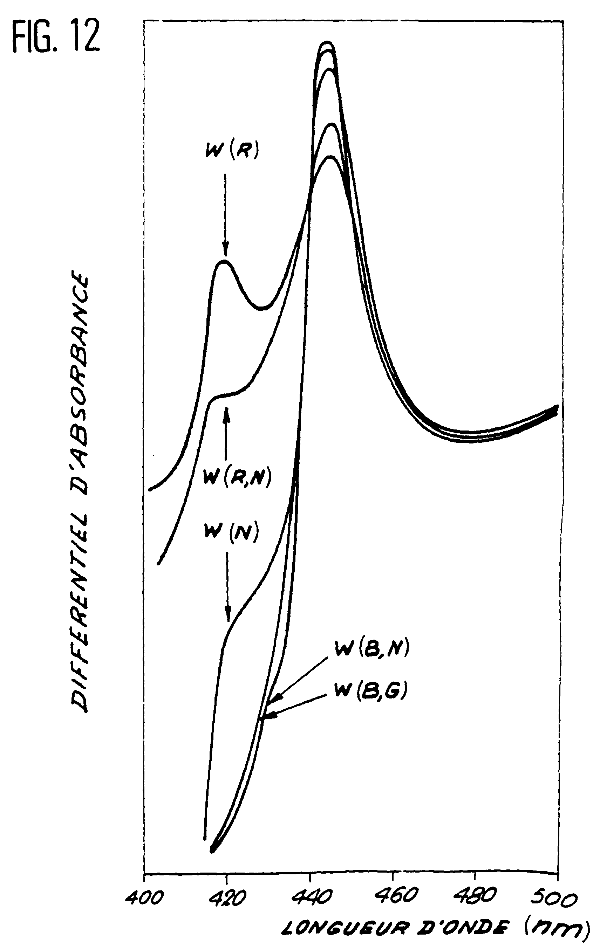

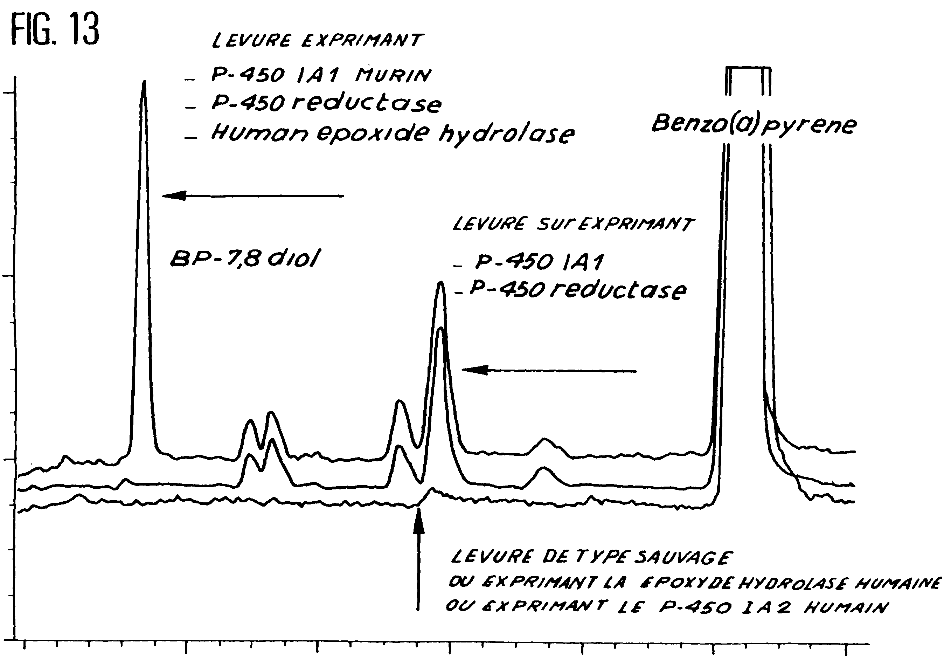

   La figure 12 démontre ce fait : dans la souche PES1-2 (notée W(N)) qui exprime un niveau sauvage de réductase, seule la présence d'un faible épaulement du spectre du P450 1A1 humain est détectable à 420 nm ce qui indique que la très grande majorité du P450 est sous forme native. Au contraire, dans la souche PES1-4 surexprimant la réductase (notée W(R)), le spectre du P450 est fortement dégradé et présente un maxima à 420nm. Dans la souche PES1-34, qui exprime le cytochrome b5 humain et qui surexprime la réductase de levure (noté W(R,B)), le spectre retrouve un aspect normal : en fait la forme dénaturée P420 devient indétectable. L'examen du tableau 3 montre que l'activité spécifique (en valeur de turn-over) du P 450 est aussi maximale dans ces conditions, et ceci malgré le niveau inférieur de réductase présent dans la souche PES-34 par rapport à celui dans la souche EP1-3. Ceci demontre que l'expression du cytochrome b5 humain dans les souches surexprimant la NADH-cytochrome P450 réductase a le double effet de stabiliser le P450 1A1 tout en permettant une activité spécifique maximale.
   Néanmoins, l'effet de la coexpression du cytochrome b5 humain est variable selon le type de cytochrome P450 considéré. Dans le cas du cytochrome P450 3A4, aucun effet de stabilisation particulier n'est observé. En revanche, un fort effet activateur est mis en évidence. Le tableau 3 indique que l'expression du cytochrome b5 seul accroît de 7 fois l'activité spécifique du P450 3A4 envers la testostérone à niveau de réductase constant. Lorsque le cytochrome b5 humain est exprimé en même temps que la réductase est surproduite, le gain d'activité atteint le chiffre considérable de 72 fois.
   En conclusion, pour les deux types de P450 testés, la production de cytochrome b5 humain apporte un avantage significatif, soit au niveau de la stabilité, soit au niveau de l'activité spécifique. Un point remarquable est que le cytochrome b5 endogène de la levure semble dépourvu de ces effets spécifiques malgré sa présence à un niveau comparable voire supérieur à celui du cytochrome b5 humain dans certaines des souches. En conséquence la nature (de mammifère) du cytochrome b5 se révèle un élément important.
   Les solutions techniques permettant dans la souche PSE1-34, de limiter les risques d'instabilité génétique tout en permettant l'expression à haut niveau sous le contrôle d'une régulation unique de trois gènes hétérologues. A savoir :
   1/ Le recours à un même promoteur inductible pour l'expression de la réductase, du cytochrome b5 et du P450 ce qui permet d'éviter les effet toxiques qui seraient liés à une surproduction permamente de ces enzymes ou à l'activité monooxygénase hétérologue résultante.
   2/ L'utilisation du même locus génomique pour l'expression de deux gènes hétérologues différents sous la forme d'un diploïde hétérozygotes. Ce qui permet de limiter les événements de recombinaison capables d'aboutir à une altération de la régulation ou des fonctions exprimées.
   3/ L'utilisation de plasmides d'expression pour le P450 utilisant le même promoteur que les gènes de réductase et de b5 intégrés mais des séquences 3'-flanquantes distinctes afin d'éviter les risques d'instabilité qui résulteraient de recombinaisons homologues entre plasmide et génome.
4.4.3. Expression de l'époxyde hydrolase humaine dans les souches PES1-5 et PES1-53 et biotransformation du benzo(a)pyrène.
   Le dosage de l'activité styrène-oxyde hydrolase dans les souches PES1-5 et PES1-53 met en évidence la formation à partir du styrène-oxyde d'environ 1 à 2 nmoles de 2-phényl-2-hydroxyéthanol par min et par mg de protéines microsomales. Cette activité est indétectable dans les autres souches de la série PESI. L'activité réductase de la souche PES1-53 est comparable à celle de la souche PES1-34. La souche PES1-53 transformée par un plasmide d'expression qui code pour le cytochrome P450 1A1 est capable, lors de l'incubation avec du benzo(a)pyrène, d'hydrolyser en benzo(a)pyrène-7,8-diol le benzo(a)pyrène-7,8-oxyde formé in situ. Ces activités sont demontrées par la figure 13 qui présente une séparation en HPLC des métabolites du benzo(a)pyrène formés lors de l'incubation de ce polluant avec les souches PES1-2, PES1-3 et PES1-53 transformées pour exprimer le cytochrome P450 lAl murin. Clairement, seule la souche PES1-53 permet la formation de benzo(a)pyrène-7,8-diol en quantité importante. Cette molécule est un intermédiaire important de la dégradation du polluant industriel carcinogène qu'est le benzo(a)pyrène.

### 5. Domaines d'application

Le domaine d'application inclut prioritairement l'expression chez la levure, des activités des membres quelconques de la superamille des cytochromes P450, et les applications pouvant en être faite. En fait l'ensemble du système décrit peut a priori être étendu à tout système d'expression hétérologue multigènique (même autre que celui du P450) permettant de réaliser une fonction complexe, telle qu'une biosynthèse hétérologue multiétape.
- des biotransformations de tout type (suivies ou non de l'extraction des produits formés) selon les isoenzymes de cytochromes P450 introduit, éventuellement des bioconversions multiétape en utilisant les extensions citées.
- La détection, l'identification, le dosage et la destruction de substances polluantes, toxiques ou cancérigènes variées (selon les types de P450 introduits). Ce type d'application pourra impliquer la coexpression de plusieurs cytochromes P450, d'enzymes relais tels que l'époxide-hydrolase, d'enzymes de désactivation tels que les glutathion-transférases et les glucoronate-transférases et de transporteurs tels que la "Multi-drugsresistance protein".
- L'élaboration de systèmes in vitro de simulation du métabolisme des médicaments chez l'homme reproduisant fidèlement les activités humaines et incluant la possibilité d'analyser qualitativement et quantitativement le rôle des différentes activités sur la nature et les niveaux des métabolites.

Les souches suivantes ont été déposées à la Collection Nationale de Cultures de Microorganismes de l'Institut Pasteur, 25 rue du Docteur Roux, 75015 Paris, le 7 juillet 1992 :

| | |
|---|---|
| PES 1-1 | sous le n° I-1230 |
| PES 1-2 | sous le n° I-1231 |
| PES 1-3 | sous le n° I-1232 |
| PES 1-4(S)* | sous le n° I-1233 |
| PES 1-4(AS)** (appelée W(B)) | sous le n° I-1234 |

| | |
|---|---|
| * : S = sens | |
| **: AS = anti-sens | |

**TABLEAU 1**

| Souches | Ploïdie | Yred loc1 | | URA3 | TRP1 |
|---|---|---|---|---|---|
| | | Locus 1 | Locus 2 | | |
| PES1-1 | hap(a) | WT | - | - | - |
| PES1-2 | hap(alpha) | WT | - | - | - |
| PES1-3 | hap(alpha) | Yred::GAL | - | + | - |
| PES1-3D | hap(a) | Del/TRP1 | - | - | - |
| PES1-3U | hap(alpha) | Yred::GAL | - | - | - |
| PES1-4 | hap(a) | DEL/b5::GAL | - | + | - |
| PES1-12 | dip | WT | WT | - | - |
| PES1-31 | dip | Yred::GAL | WT | + | - |
| PES1-34 | dip | Yred::GAL | Del/b5::GAL | + | - |
| PES1-42 | dip | WT | Del/b5::GAL | + | - |
| PES1-5 | hap(al) | Del/HEH::GAL | - | + | - |
| PES1-53 | dip | Yred::GAL | Del/HEH::GAL | + | - |
| Légende : hap : haploïde ; dip : diploïde b5::GAL : cytochrome b5 humain sous le contrôle transcriptionnel de GAL10-CYC1 HEH::GAL : l'époxyde hydrolase humaine sous le contrôle transcriptionnel de GAL10-CYC1 Del : Délété WT : type sauvage Yred::GAL : ORF Yred sous le contrôle transcriptionnel du promoteur GAL10-CYC1 | | | | | |

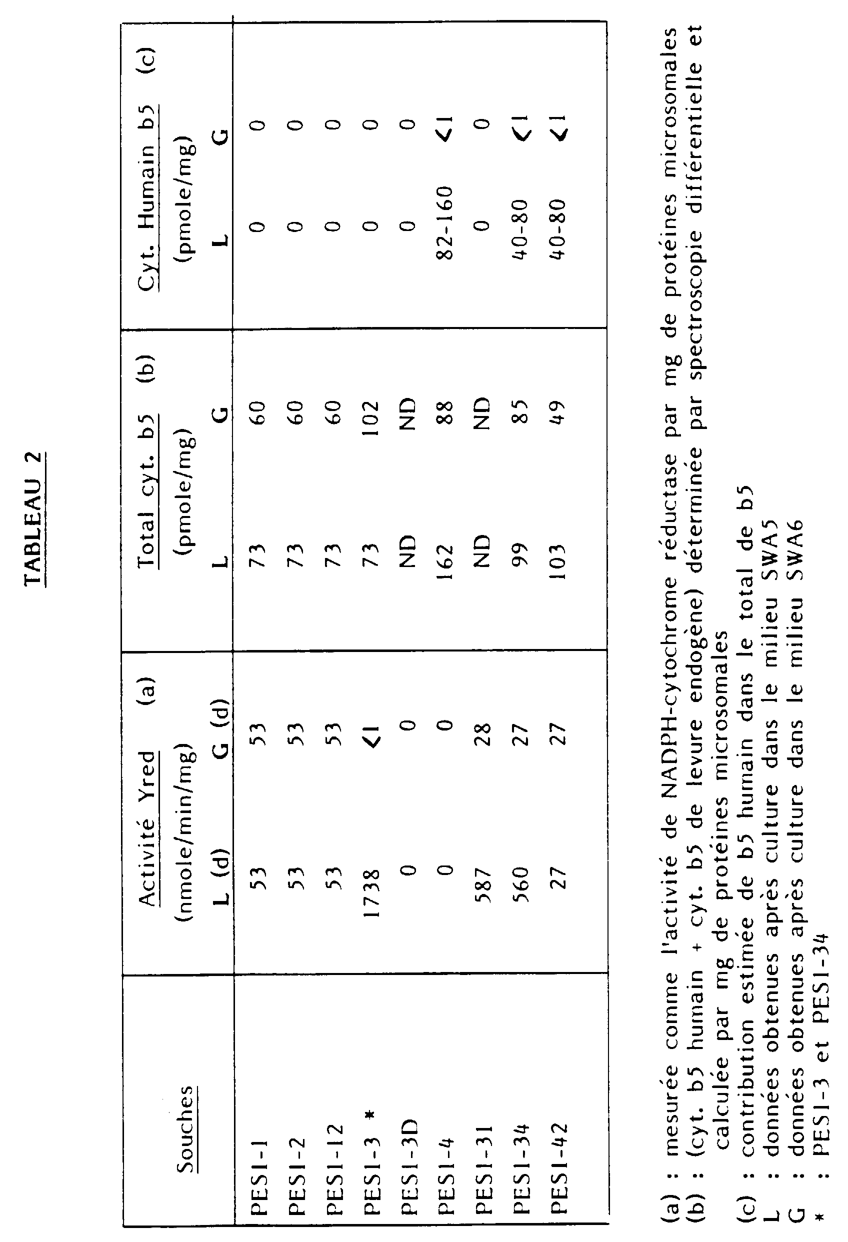

**TABLEAU 3**

| Souches | P450 1A1 | P450 1A1 | P450 3A4 |
|---|---|---|---|
| | (Murin) (a) | (Humain) (b) | (Humain) (c) |
| PES1-2 | 1 | 1 | 1 |
| PES1-3 | 9,3 | 4,4 | 62 |
| PES1-4 | 0 | 0 | 0 |
| PES1-31 | - | 3,5 | 44 |
| PES1-34 | - | 5 | 73 |
| PES1-42 | - | 0,5 | 7 |
| PES1-53 | - | 3,5 | - |

| | | | |
|---|---|---|---|
| Activités des cytochromes P450 (a), (b) : activité testée comme éthoxyrésurufin-O-déethylase, la valeur absolue pour PES1-2 est (a) : 1,5 nmole/nmole de P450/min ; (b) : 3,2 nmole P450/min ; | | | |
| (c) : testée en tant qu'activité testostérone 6-béta-hydroxylase, la valeur absolue pour PES1-2 est 31 pmole/nmole de P450/min | | | |

## Revendications (Revendications pour l'(les) Etat(s) contractant(s) suivant(s): AT, BE, CH, LI, DE, DK, FR, GB, IT, LU, NL, SE)

1. Souche de levure dans le génome chromosomique de laquelle sont intégrés de façon stable un ou plusieurs gènes hétérologues et permettant leur expression, caractérisée en ce que :
1 - la souche de levure est une souche diploïde dont les allèles sont isogéniques à l'exception des loci qui portent les gènes hétérologues et du locus sexuel,
2 - les loci hétérozygotes sont dépourvus d'allèle portant un gène sauvage et
3 -les gènes hétérologues sont placés sous le contrôle d'un promoteur inductible et régulable.

2. Souche selon la revendication 1 caractérisée en ce que chaque locus hétérozygote ne comporte pas d'homologie de séquence entre les deux allèles qui contiennent des gènes hétérologues ou artificiels.

3. Souche selon la revendication 1 caractérisée en ce que les séquences homologues entre deux allèles dans chaque hétérozygote sont orientées inversement.

4. Souche selon l'une des revendications 1 à 3 caractérisée en ce que les unités d'expression de(s) gène(s) hétérologue(s) comporte(nt) un marqueur sélectionnable lorsque le(s)dit(s) gène(s) hétérologue(s), constitue(nt) un facteur négatif pour la vitesse de croissance de la souche.

5. Souche selon l'une des revendications 1 à 4 caractérisée en ce qu'on utilise le même locus génomique sur chaque allèle pour l'expression de deux gènes hétérologues différents, lesquels ainsi forment un locus diploïde hétérozygote.

6. Souche selon l'une des revendications 1 à 5 caractérisée en ce que les gènes hétérologues intégrés codent pour des facteurs constitutifs d'une chaîne de bioconversion multi-étape ou constituant une fonction complexe.

7. Souche selon l'une des revendications 1 à 6 dans le génome chromosomique de laquelle sont intégrés de manière stable un gène de la NADPH-cytochrome P450-réductase de levure ou hétérologue et un gène de cytochrome b5 hétérologue.

8. Souche selon la revendication 7 caractérisée en ce que le gène de la NADPH-cytochrome P450-réductase est intégré dans le locus de la NADPH-cytochrome P450 réductase endogène.

9. Souche selon l'une des revendications 1 à 5 caractérisée en ce que elle co-exprime le gène de l'époxyde hydrolase humaine et le gène de la NADPH-cytochrome P450-réductase dans le génome chromosomique de laquelle ils sont intégrés.

10. Souche selon l'une des revendications 1 à 9 caractérisée en ce qu'il s'agit d'une souche de Saccharomyces cerevisiae.

11. Procédé d'expression chez la levure de facteurs constituant une fonction complexe, notamment une chaîne de bioconversion multi-étape, dont l'activité principale est liée à un premier facteur hétérologue donné caractérisé en ce qu'on transforme une souche de levure selon l'une des revendications 1 à 10, ladite souche co-exprimant d'autres facteurs hétérologues contribuant à la fonction complexe et dans le génome chromosomique de laquelle les gènes correspondants aux dits autres facteurs sont intégrés, ladite souche étant transformée avec un plasmide portant une cassette d'expression dudit premier gène hétérologue.

12. Procédé d'expression chez la levure de l'activité monoxygénase de cytochrome P450 hétérologue caractérisé en ce qu'on transforme une souche de levure selon l'une des revendications I à 10 co-exprimant de la NADHP-cytochrome-P450 réductase et du cytochrome b5 dans le génome chromosomique de laquelle sont intégrés les gènes de la NADPH-cytochrome-P450 réductase et du cytochrome b5, à l'aide d'un plasmide portant une cassette d'expression du gène du cytochrome P450 hétérologue.

13. Procédé selon la revendication 12 caractérisé en ce que le cytochrome b5 est de la même espèce que le cytochrome P450.

14. Ensemble de souches de levures conformes à l'une des revendications 1 à 8 et 10 permettant l'expression de cytochromes P450 hétérologues, chaque élément de cet ensemble pouvant être transformé par des plasmides portant une cassette d'expression d'un cytochrome P450 hétérologue conformément au procédé de la revendication 12, les différentes souches de cet ensemble ayant intégré dans le génome de leurs chromosomes les gènes de la NADPH-cytochrome P450-réductase et du cytochrome b5, gènes dont les niveaux d'expression respectifs sont variables entre les différentes souches de sorte que cet ensemble permet de déterminer la souche optimale pour l'expression de chaque cytochrome P450 spécifique.

## Revendications (Revendications pour l'(les) Etat(s) contractant(s) suivant(s): ES)

1. Procédé permettant l'expression d'un ou plusieurs gènes hétérologues chez une souche de levure dans le génome chromosomique de laquelle sont intégrés de façon stable un ou plusieurs gènes hétérologues,caractérisé en ce que :
1) la souche de levure est une souche diploïde dont les allèles sont isogéniques à l'exception des loci qui portent les gènes hétérologues et du locus sexuel,
2) les loci hétérozygotes sont dépourvus d'allèle portant un gène sauvage, et
3) on place les gènes hétérologues sous le contrôle d'un promoteur inductible et régulable.

2. Procédé selon la revendication 1 caractérisé en ce que chaque locus hétérozygote ne comporte pas d'homologie de séquence entre les deux allèles qui contiennent des gènes hétérologues ou artificiels.

3. Procédé selon la revendication 1 caractérisé en ce que les séquences homologues entre deux allèles dans chaque hétérozygote sont orientées inversement.

4. Procédé selon l'une des revendications 1 à 3 caractérisé en ce que les unités d'expression de(s) gène(s) hétérologue(s) comportent un marqueur sélectionnable lorsque le(s)dit(s) gène(s) hétérologue(s), constitue(nt) un facteur négatif pour la vitesse de croissance de la souche.

5. Procédé selon l'une des revendications 1 à 4 caractérisé en ce qu'on utilise le même locus génomique sur chaque allèle pour l'expression de deux gènes hétérologues différents, lesquels ainsi forment un locus diploïde hétérozygote.

6. Procédé selon l'une des revendications 1 à 5 caractérisé en ce que les gènes hétérologues intégrés codent pour des facteurs constitutifs d'une chaîne de bioconversion multi-étape ou constituant une fonction complexe.

7. Procédé selon l'une des revendications 1 à 6 caractérisé en ce qu'un gène de la NADPH-cytochrome P450-réductase de levure ou hétérologue et un gène de cytochrome b5 hétérologue sont intégrés de manière stable dans le génome chromosomique de ladite souche de levure.

8. Procédé selon la revendication 7 caractérisé en ce que le gène de la NADPH-cytochrome P450-réductase est intégré dans le locus de la NADPH-cytochrome P450-réductase endogène.

9. Procédé selon l'une des revendications 1 à 5 caractérisé en ce que la souche, dans le génome chromosomique de laquelle le gène de l'époxyde hydrolase humaine et le gène de la NADPH-cytochrome P450-réductase sont intégrés, coexprime lesdits gènes.

10. Procédé selon l'une des revendications 1 à 9 caractérisé en ce que la souche de levure est une souche de Saccharomyces cerevisiae.

11. Procédé selon l'une des revendications 1 à 10 pour l'expression chez la levure, de facteurs constituant une fonction complexe, notamment une chaîne de bioconversion multi-étape dont l'activité principale est liée à un premier facteur hétérologue donné, selon lequel ladite souche de levure dans le génome chromosomique de laquelle les gènes correspondants auxdits autres facteurs sont intégrés, co-exprime d'autres facteurs hétérologues contribuant à la fonction complexe, ladite souche étant transformée avec un plasmide portant une cassette d'expression dudit premier gène hétérologue.

12. Procédé selon l'une des revendications 1 à 10 pour l'expression chez la levure de l'activité monoxygénase de cytochrome P450 hétérologue, selon lequel ladite souche de levure dans le génome chromosomique de laquelle sont intégrés les gènes de la NADPH-cytochrome P450-réductase et du cytochrome b5 coexprime de la NADPH-cytochrome P450-réductase et du cytochrome b5, ladite souche étant transformée, à l'aide d'un plasmide portant une cassette d'expression du gène du cytochrome P450 hétérologue.

13. Procédé selon la revendication 12 caractérisé en ce que le cytochrome b5 est de la même espèce que le cytochrome P450.

14. Procédé permettant l'expression de cytochrome P450 hétérologue chez un ensemble de souches de levures telles que définies à l'une des revendications 1 à 8 et 10, chaque élément de cet ensemble pouvant être transformé par des plasmides portant une cassette d'expression d'un cytochrome P450 hétérologue, les différentes souches de cet ensemble ayant intégré dans le génome de leurs chromosomes les gènes de la NADPH-cytochrome P450-réductase et du cytochrome b5, gènes dont les niveaux d'expression respectifs sont variables entre les différentes souches de sorte que cet ensemble permet de déterminer la souche optimale pour l'expression de chaque cytochrome P450 spécifique.

## Patentansprüche (Patentansprüche für folgende(n) Vertragsstaat(en): AT, BE, CH, LI, DE, DK, FR, GB, IT, LU, NL, SE)

1. Hefestamm, in dessen Chromosomen-Genom ein oder mehrere heterologe Gene auf stabile Weise integriert sind und der ihre Expression erlaubt, dadurch gekennzeichnet, daß
1) der Hefestamm ein diploider Stamm ist, dessen Allele isogen sind mit Ausnahme der Loki, die heterologene Gene tragen, und des sexuellen Lokus,
2) die heterozygoten Loki frei von einem Allel sind, das ein Wild-Gen trägt, und
3) die heterologen Gene unter der Kontrolle eines induzierbaren und regulierbaren Promotors stehen.

2. Stamm nach Anspruch 1, dadurch gekennzeichnet, daß jeder heterozygote Lokus keine Sequenz-Homologie zwischen den beiden Allelen aufweist, die heterologe oder künstliche Gene enthalten.

3. Stamm nach Anspruch 1, dadurch gekennzeichnet, daß die homologen Sequenzen zwischen zwei Allelen in jedem Heterozygoten umgekehrt (entgegengesetzt) orientiert sind.

4. Stamm nach einem der Ansprüche 1 bis 3, dadurch gekennzeichnet, daß die Einheiten zur Expression des (der) heterologen Gens (Gene) einen selektionierbaren Marker aufweisen, wenn das (die) heterologe(n) Gen(e) einen negativen Faktor für die Wachstumsgeschwindigkeit des Stammes darstellt (darstellen).

5. Stamm nach einem der Ansprüche 1 bis 4, dadurch gekennzeichnet, daß man auf jedem Allel den gleichen Genom-Lokus zur Expression von zwei verschiedenen heterologen Genen verwendet, die auf diese Weise einen heterozygoten diploiden Lokus bilden.

6. Stamm nach einem der Ansprüche 1 bis 5, dadurch gekennzeichnet, daß die integrierten heterologen Gene für konstitutive Faktoren einer Mehrstufen-Bioumwandlungs-Kette codieren oder eine komplexe Funktion darstellen.

7. Stamm nach einem der Ansprüche 1 bis 6, in dessen Chromosomen-Genom ein Gen der NADPH-Cytochrom P450-Reduktase von Hefe oder ein Heterologon und ein heterologes Gen von Cytochrom b5 auf stabile Weise integriert sind.

8. Stamm nach Anspruch 7, dadurch gekennzeichnet, daß das Gen der NADPH-Cytochrom P450-Reduktase in den Lokus der endogenen NADPH-Cytochrom P450-Reduktase integriert ist.

9. Stamm nach einem der Ansprüche 1 bis 5, dadurch gekennzeichnet, daß er das Gen der Human-Epoxid-Hydrolase und das Gen der NADPH-Cytochrom P450-Reduktase, die in sein Chromosomen-Genom integriert sind, coexprimiert.

10. Stamm nach einem der Ansprüche 1 bis 9, dadurch gekennzeichnet, daß es sich dabei um einen Stamm von Saccharomyces cerevisiae handelt.

11. Verfahren zur Expression von Faktoren, die eine komplexe Funktion, insbesondere eine Mehrstufen-Bioumwandlungs-Kette darstellen, deren Hauptaktivität an einen gegebenen heterologen ersten Faktor gebunden ist, bei der Hefe, dadurch gekennzeichnet, daß man einen Hefestamm nach einem der Ansprüche 1 bis 10, der weitere (andere) heterologe Faktoren coexprimiert, die zur komplexen Funktion beitragen und in dessen Chromosomen-Genom die den anderen (weiteren) Faktoren entsprechenden Gene integriert sind, transformiert, wobei der genannte Stamm mit einem Plasmid transformiert wird, das eine Kassette zur Expression des genannten ersten heterologen Gens trägt.

12. Verfahren zur Expression der Monooxygenase-Aktivität von heterologem Cytochrom P450 bei der Hefe, dadurch gekennzeichnet, daß man einen Hefestamm nach einem der Ansprüche 1 bis 10, der NADHP-Cytochrom-P450-Reduktase und Cytochrom b5 coexprimiert, in dessen Chromosomen-Genom die Gene der NADPH-Cytochrom-P450-Reduktase und des Cytochroms b5 integriert sind, mit Hilfe eines Plasmids transformiert, das eine Kassette zur Expression des heterologen Gens von Cytochrom P450 trägt.

13. Verfahren nach Anspruch 12, dadurch gekennzeichnet, daß das Cytochrom b5 von der gleichen Art ist wie das Cytochrom P450.

14. Ansammlung (Gruppe) von Hefestämmen nach einem der Ansprüche 1 bis 8 und 10, welche die Expression von heterologen Cytochromen P450 erlauben, wobei jedes Element dieser Ansammlung (Gruppe) durch Plasmide, die eine Kassette zur Expression eines heterologen Cytochroms P450 tragen, transformiert werden kann nach dem Verfahren des Anspruchs 12, wobei die verschiedenen Stämme dieser Ansammlung (Gruppe) in dem Genom ihrer Chromosomen die Gene der NADPH-Cytochrom-P450-Reduktase und des Cytochroms b5 integriert enthalten, deren jeweilige Expressions-Niveaus zwischen den verschiedenen Stämmen in der Weise variabel sind, daß diese Ansammlung (Gruppe) die Bestimmung des Stammes erlaubt, der optimal ist für die Expression jedes spezifischen Cytochroms P450.

## Patentansprüche (Patentansprüche für folgende(n) Vertragsstaat(en): ES)

1. Verfahren, das die Expression eines oder mehrerer heterologer Gene bei einem Hefestamm erlaubt, in dessen Chromosomen-Genom ein oder mehrere heterologene Gene auf stabile Weise integriert sind, dadurch gekennzeichnet, daß
1) der Hefestamm ein diploider Stamm ist, dessen Allele isogen sind mit Ausnahme der Loki, die heterologene Gene tragen, und des sexuellen Lokus,
2) die heterozygoten Loki frei von einem Allel sind, das ein Wild-Gen trägt, und
3) die heterologen Gene unter der Kontrolle eines induzierbaren und regulierbaren Promotors stehen.

2. Verfahren nach Anspruch 1, dadurch gekennzeichnet, daß jeder heterozygote Lokus keine Sequenz-Homologie zwischen den beiden Allelen aufweist, die heterologe oder künstliche Gene enthalten.

3. Verfahren nach Anspruch 1, dadurch gekennzeichnet, daß die homologen Sequenzen zwischen zwei Allelen in jedem Heterozygoten umgekehrt (entgegengesetzt) orientiert sind.

4. Verfahren nach einem der Ansprüche 1 bis 3, dadurch gekennzeichnet, daß die Einheiten zur Expression des (der) heterologen Gens (Gene) einen selektionierbaren Marker aufweisen, wenn das (die) heterologe(n) Gen(e) einen negativen Faktor für die Wachstumsgeschwindigkeit des Stammes darstellt (darstellen).

5. Verfahren nach einem der Ansprüche 1 bis 4, dadurch gekennzeichnet, daß man auf jedem Allel den gleichen Genom-Lokus zur Expression von zwei verschiedenen heterologen Genen verwendet, die auf diese Weise einen heterozygoten diploiden Lokus bilden.

6. Verfahren nach einem der Ansprüche 1 bis 5, dadurch gekennzeichnet, daß die integrierten heterologen Gene für konstitutive Faktoren einer Mehrstufen-Bioumwandlungs-Kette codieren oder eine komplexe Funktion darstellen.

7. Verfahren nach einem der Ansprüche 1 bis 6, dadurch gekennzeichnet, daß ein Gen der NADPH-Cytochrom P450-Reduktase von Hefe oder ein Heterologon und ein heterologes Gen von Cytochrom b5 auf stabile Weise in das Chromosomen-Genom des genannten Hefestammes integriert werden.

8. Verfahren nach Anspruch 7, dadurch gekennzeichnet, daß das Gen der NADPH-Cytochrom P450-Reduktase in den Lokus der endogenen NADPH-Cytochrom P450-Reduktase integriert ist.

9. Verfahren nach einem der Ansprüche 1 bis 5, dadurch gekennzeichnet, daß der Stamm, in dessen Chromosomen-Genom das Gen der Human-Epoxid-Hydrolase und das Gen der NADPH-Cytochrom P450-Reduktase integriert sind, die genannten Gene coexprimiert.

10. Verfahren nach einem der Ansprüche 1 bis 9, dadurch gekennzeichnet, daß der Hefestamm ein Stamm von Saccharomyces cerevisiae ist.

11. Verfahren nach einem der Ansprüche 1 bis 10 zur Expression von Faktoren, die eine komplexe Funktion, insbesondere eine Mehrstufen-Bioumwandlungs-Kette darstellen, deren Hauptaktivität an einen gegebenen heterologen ersten Faktor gebunden ist, bei der Hefe, bei dem der genannte Hefestamm, in dessen Chromosomen-Genom die den genannten anderen (weiteren) Faktoren entsprechenden Gene integriert sind, andere (weitere) heterologe Faktoren coexprimiert, die zu der komplexen Funktion beitragen, wobei der genannte Stamm mit einem Plasmid transformiert wird, das eine Kassette zur Expression des genannten heterologen ersten Gens trägt.

12. Verfahren nach einem der Ansprüche 1 bis 10 zur Expression der Monooxygenase-Aktivität von heterologem Cytochrom P450 bei der Hefe, bei dem der genannte Hefestamm, in dessen Chromosomen-Genom die Gene der NADHP-Cytochrom-P450-Reduktase und des Cytochroms b5 integriert sind, die NADPH-Cytochrom-P450-Reduktase und das Cytochrom b5 coexprimiert, wobei der genannte Stamm mit Hilfe eines Plasmids transformiert wird, das eine Kassette zur Expression des Gens des heterologen Gens von Cytochrom P450 trägt.

13. Verfahren nach Anspruch 12, dadurch gekennzeichnet, daß das Cytochrom b5 von der gleichen Art ist wie das Cytochrom P450.

14. Verfahren, das die Expression von heterologem Cytochrom P450 bei einer Ansammlung (Gruppe) von Hefestämmen nach einem der Ansprüche 1 bis 8 und 10 erlaubt, wobei jedes Element dieser Ansammlung (Gruppe) durch Plasmide, die eine Kassette zur Expression eines heterologen Cytochroms P450 tragen, transformiert werden kann, wobei die verschiedenen Stämme dieser Ansammlung (Gruppe) in ihrem Chromosomen-Genom die Gene der NADPH-Cytochrom-P450-Reduktase und des Cytochroms b5 integriert enthalten, deren jeweilige Expressions-Niveaus zwischen den verschiedenen Stämmen in der Weise variabel sind, daß diese Ansammleung (Gruppe) die Bestimmung des Stammes erlaubt, der für die spezifische Expression des jeweiligen Cytochroms P450 optimal ist.

## Claims (Claims for the following Contracting State(s): AT, BE, CH, LI, DE, DK, FR, GB, IT, LU, NL, SE)

1. Yeast strain in the chromosomal genome of which one or more heterologous genes are stably integrated, and which permits their expression, characterized in that:
1 - the yeast strain is a diploid strain in which the alleles are isogenic, with the exception of the loci which carry the heterologous genes and of the mating-type locus,
2 - the heterozygous loci lack an allele carrying a wild-type gene, and
3 - the heterologous genes are placed under the control of an inducible and regulable promoter.

2. Strain according to Claim 1, characterized in that each heterozygous locus possesses no sequence homology between the two alleles which contain heterologous or artificial genes.

3. Strain according to Claim 1, characterized in that the sequences which are homologous between two alleles in each heterozygote are oriented in opposite directions.

4. Strain according to one of Claims 1 to 3, characterized in that the units for the expression of the heterologous gene(s) contain a selectable marker when the said heterologous gene(s) constitute(s) a negative factor for the rate of growth of the strain.

5. Strain according to one of Claims 1 to 4, characterized in that the same genomic locus on each allele is used for the expression of two different heterologous genes, which thus form a heterozygous diploid locus.

6. Strain according to one of Claims 1 to 5, characterized in that the integrated heterologous genes code for factors that constitute a multistep bioconversion chain or constituting a complex function.

7. Strain according to one of Claims 1 to 6, in the chromosomal genome of which a yeast or heterologous NADPH-cytochrome P450 reductase gene and a heterologous cytochrome b5 gene are stably integrated.

8. Strain according to Claim 7, characterized in that the NADPH-cytochrome P450 reductase gene is integrated in the endogenous NADPH-cytochrome P450 reductase locus.

9. Strain according to one of Claims 1 to 5, characterized in that it coexpresses the human epoxide hydrolase gene and the NADPH-cytochrome P450 reductase gene in the chromosomal genome of which they are integrated.

10. Strain according to one of Claims 1 to 9, characterized in that the strain in question is a Saccharomyces cerevisiae strain.

11. Method of expression in yeast of factors constituting a complex function, in particular a multistep bioconversion chain, the main activity of which is linked to a first given heterologous factor, characterized in that a yeast strain according to one of Claims 1 to 10 is transformed, the said strain coexpressing other heterologous factors contributing to the complex function, and in the chromosomal genome of which the genes corresponding to the said other factors are integrated, the said strain being transformed with a plasmid carrying a cassette for the expression of the said first heterologous gene.

12. Method of expression in yeast of the monooxygenase activity of heterologous cytochrome P450, characterized in that a yeast strain according to one of Claims 1 to 10 coexpressing NADPH-cytochrome P450 reductase and cytochrome b5, in the chromosomal genome of which the NADPH-cytochrome P450 reductase and cytochrome b5 genes are integrated, is transformed using a plasmid carrying a cassette for the expression of the heterologous cytochrome P450 gene.

13. Method according to Claim 12, characterized in that the cytochrome b5 is of the same species as the cytochrome P450.

14. Set of yeast strains according to one of Claims 1 to 8 and 10 which permit the expression of heterologous cytochromes P450, it being possible for each member of this set to be transformed with plasmids carrying a cassette for the expression of a heterologous cytochrome P450 according to the method of Claim 12, the different strains of this set having integrated the NADPH-cytochrome P450 reductase and cytochrome b5 genes in the genome of their chromosomes, the respective levels of expression of these genes being variable between the different strains so that this set enables the optimal strain for the expression of each specific cytochrome P450 to be determined.

## Claims (Claims for the following Contracting State(s): ES)

1. Method which permits the expression of one or more heterologous genes in a yeast strain in the chromosomal genome of which one or more heterologous genes are stably integrated, characterized in that:
1) the yeast strain is a diploid strain in which the alleles are isogenic, with the exception of the loci which carry the heterologous genes and of the mating-type locus,
2) the heterozygous loci lack an allele carrying a wild-type gene, and
3) the heterologous genes are placed under the control of an inducible and regulable promoter.

2. Method according to Claim 1, characterized in that each heterozygous locus possesses no sequence homology between the two alleles which contain heterologous or artificial genes.

3. Method according to Claim 1, characterized in that the sequences which are homologous between two alleles in each heterozygote are oriented in opposite directions.

4. Method according to one of Claims 1 to 3, characterized in that the units for the expression of the heterologous gene(s) contain a selectable marker when the said heterologous gene(s) constitute(s) a negative factor for the rate of growth of the strain.

5. Method according to one of Claims 1 to 4, characterized in that the same genomic locus on each allele is used for the expression of two different heterologous genes, which thus form a heterozygous diploid locus.

6. Method according to one of Claims 1 to 5, characterized in that the integrated heterologous genes code for factors that constitute a multistep bioconversion chain or constituting a complex function.

7. Method according to one of Claims 1 to 6, characterized in that a yeast or heterologous NADPH-cytochrome P450 reductase gene and a heterologous cytochrome b5 gene are stably integrated in the chromosomal genome of the said yeast strain.

8. Method according to Claim 7, characterized in that the NADPH-cytochrome P450 reductase gene is integrated in the endogenous NADPH-cytochrome P450 reductase locus.

9. Method according to one of Claims 1 to 5, characterized in that the strain, in the chromosomal genome of which the human epoxide hydrolase gene and the NADPH-cytochrome P450 reductase gene are integrated, coexpresses the said genes.

10. Method according to one of Claims 1 to 9, characterized in that the yeast strain is a Saccharomyces cerevisiae strain.

11. Method according to one of Claims 1 to 10 for the expression in yeast of factors constituting a complex function, in particular a multistep bioconversion chain, the main activity of which is linked to a first given heterologous factor, according to which the said yeast strain, in the chromosomal genome of which the genes corresponding to the said other factors are integrated, coexpresses other heterologous factors contributing to the complex function, the said strain being transformed with a plasmid carrying a cassette for the expression of the said first heterologous gene.

12. Method according to one of Claims 1 to 10 for the expression in yeast of the monooxygenase activity of heterologous cytochrome P450, according to which the said yeast strain, in the chromosomal genome of which the NADPH-cytochrome P450 reductase and cytochrome b5 genes are integrated, coexpresses NADPH-cytochrome P450 reductase and cytochrome b5, the said strain being transformed using a plasmid carrying a cassette for the expression of the heterologous cytochrome P450 gene.

13. Method according to Claim 12, characterized in that the cytochrome b5 is of the same species as the cytochrome P450.

14. Method which permits the expression of heterologous cytochrome P450 in a set of yeast strains as defined in one of Claims 1 to 8 and 10, it being possible for each member of this set to be transformed with plasmids carrying a cassette for the expression of a heterologous cytochrome P450, the different strains of this set having integrated the NADPH-cytochrome P450 reductase and cytochrome b5 genes in the genome of their chromosomes, the respective levels of expression of these genes being variable between the different strains so that this set enables the optimal strain for the expression of each specific cytochrome P450 to be determined.
